# EUROPEAN PATENT SPECIFICATION

(11) **EP 0 178 867 B1**
(45) Date of publication and mention of the grant of the patent: **12.01.1994**
(21) Application number: 85307311.2
(22) Date of filing: 11.10.1985
(51) Int. Cl.: C12N 15/16, C07K 13/00, C12P 21/02, C12N 5/00, A61K 37/24, A61K 47/00, C07K 15/00, G01N 33/74

(54) **Novel hormones and methods for making, using and assaying same**
Hormone und Verfahren zur Herstellung, Verwendung und Prüfung derselben
Hormones et procédés de production, utilisation et essai de celles-ci

(30) Priority: 19.10.1984 US 663097; 08.03.1985 US 709959
(43) Date of publication of application: 23.04.1986
(73) Proprietor: GENENTECH, INC., South San Francisco California 94080 (US)
(72) Inventor: Seeburg, Peter Horst, San Francisco California 94122 (US); Adelman, John Peter, Eugene Oregon 97403 (US)
(74) Representative: Armitage, Ian Michael

(56) References cited:
- EP-A- 0 036 776
- EP-A- 0 070 186
- NATURE, vol. 311, no. 5987, 18th October 1984, pages 666-668, Reading, Berks,GB; P.H. SEEBURG et al.: "Characterization of cDNA for precursor of human
- luteinizing hormone releasing hormone"
- Idem
- NATURE, vol. 316, no. 6028, 8th August 1985, pages 511-517, Reading, Berks, GB;K. NIKOLICS et al.: "A prolactin-inhibiting factor within the precursor forhuman gonadotropin-releasing hormone"
- NATURE, vol. 316, no. 6028, 8th August 1985, pages 542-545, Reading, Berks, GB;H.S. PHILLIPS et al.: "Immunocytochemical localization in rat brain of aprolactin release-inhibiting sequence of gonadotropin-releasing hormoneprohormone"
- T158/91,T296/87, T2/83
- Ann. Rev. Biochem. 1984, 53:665-715

## Description

This application relates to the identification and synthesis of novel hormones. In particular it is concerned with hormones involved in the biochemistry of reproduction in mammals.

Human reproduction is controlled by the hypothalamic-pituitary-gonadal axis appearing early in fetal development. Luteinizing hormone releasing hormone (LHRH), also termed gonadotropin releasing hormone (GnRH), is a decapeptide of known structure and represents a key molecule in this control circuit. It is produced by hypothalamic neurons, secreted in a pulsatile manner into the capillary plexus of the median eminence and effects the release of luteinizing hormone and follicle stimulating hormone from gonadotrophic cells in the anterior pituitary. LHRH or LHRH-like immunoreactivity has also been found in gonadal tissue placenta, [G. Khodr et al., "Science" 207: 315-317 (1980) and J. Gautron et al., "Mol. Cell. Endocrinology" 24: 1-16 (1981)] and the central nervous system, thereby suggesting additional functions of the peptide. Its presence in the latter, as well as the behavioral effects of exogenously administered LHRH, have led researchers to postulate the involvement of this peptide as a true transcriber in the control of reproductive behavior [R. Moss, "Fed. Proc." 36: 1978-1983 (1977)].

The existence of precursor forms of LHRH were suggested by chromatographic studies of hypothalamic and placental extracts. See J. Gautron, Id., and R. Millar et al., "Biochem. Biophys. Res. Commun." 74: 720-726 (1977). However, these forms were poorly characterized, present in impure mixtures and available only in extremely small quantities.

Nature 311, 666-668 (1984) discloses a nucleic acid sequence and deduced amino acid sequence of a putative LHRH precursor.

It has long been recognized that prolactin (PRL) secretion from the anterior pituitary is predominantly under inhibitory control. Although dopamine is known to exert such an inhibitory effect, it has been convincingly demonstrated that dopamine cannot be solely responsible for overall PRL inhibition. The existence of a major PRL-inhibitory factor (PIF) of peptidic nature has been invoked, but such a factor has not yet been characterized from hypothalamic extracts. Attempts to isolate hypothalamic PIF have shown that the areas containing dopamine-free PIF activity were located in the mediobasal hypothalamus and the organum vasculosum lamina terminal is [A. Enjalbert et al., "Neuroendocrinology" 24: 147-161 (1977)], regions which abound in LHRH-producing and secreting neurons. Preliminary purification studies from porcine and ovine hypothalamic tissue allowed size estimates for this activty of between 2000 and 8000 daltons [A. Enjalbert et al., Id.; A.P.S. Dharimal et al., "Endocrinology" 82: 1236-1241 (1968); and T. Greibrokk et al., "Biochem. Biophys. Res. Commun." 59: 704-709 (1974)]. Despite the time that has passed since these publications, the proteins responsible for PIF activity remains uncharacterized and unavailable in pure form.

It is an object of the invention to identify a polypeptide capable of PIF activity, to synthesize such a polypeptide in recombinant cell culture in large quantities and thereafter to obtain same in purified form.

It is another object to employ such a polypeptide in analytical procedures, in the therapeutic modulation of reproductive cycles and in the treatment of prolactin dependent tumors.

These and other objects will be apparent to the ordinary artisan from consideration of this specification as a whole.

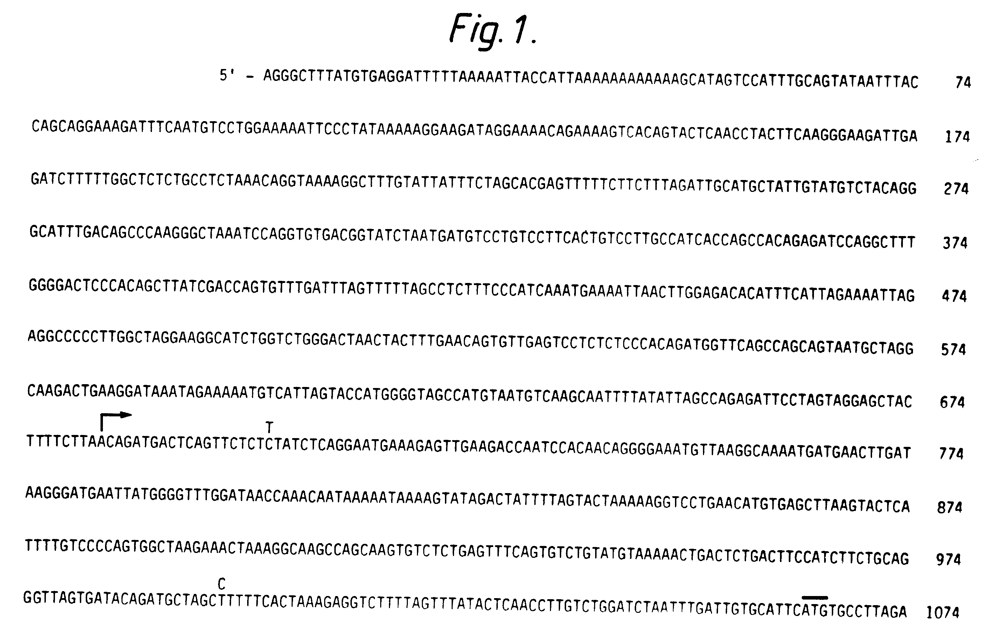

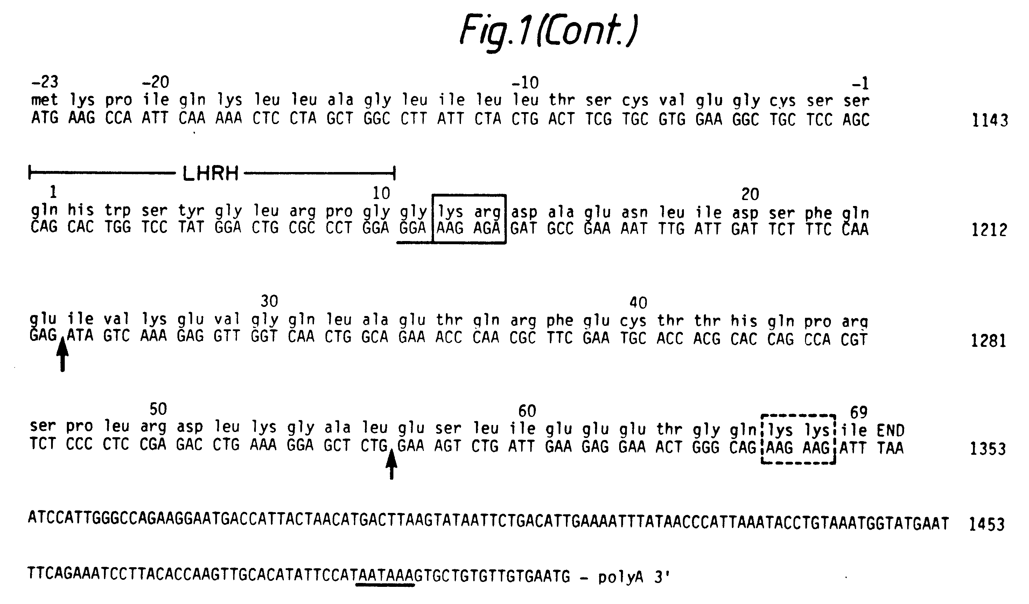

DNA encoding an LHRH precursor has been identified in studies of the human genome (Fig. 1). This precursor, preproLHRH, contains in downstream order a eukaryotic signal peptide, LHRH and a C-terminal polypeptide (CTP). The processed fusion of LHRH and CTP is termed proLHRH. ProLHRH is believed to be proteolytically processed in vivo at one, and possibly two, protease cleavage sites, thereby ultimately yielding LHRH and CTP. CTP is defined to include the 53 amino acid polypeptide shown at residues 14 to 66 in Fig. 1. CTP having the additional three carboxy terminal residues Lys Lys Ile is termed GAP (GnRH-associated peptide).

By virtue of the disclosures herein it is now possible to prepare a polypeptide which is free of LHRH and comprising a CTP amino acid sequence, CTP being defined herein to include CTP as well as mutants or fragments thereof. These cell-free polypeptides include fusions of CTP with eukaryotic proteins, other than with LHRH, for example with polypeptides other than the signal polypeptide ordinarily expressed with proLHRH in vivo, e.g. bacterial signals or bacterially secreted proteins. The polypeptides may be administered to mammals in formulations with physiologically acceptable carriers or timed-release agents in order to modulate reproductive cycles or treat PRL-dependent tumors, they may be label led with detectable markers for use in immunoassays, or they may be employed as immunogens to raise anti-CTP antisera for diagnosis, for purification of CTP or its fusions such as proLHRH, or for derepression of PRL secretion in dairy animals.

These polypeptides, including CTP, are synthesized by conventional organic chemical techniques or, preferably, by recombinant DNA methods. The latter comprises transforming a host cell with a vector known to encode the polypeptide followed by culturing the cells and, ordinarily, recovering the polypeptide from the cell.

### Brief Description of the Drawings

Fig. 1 discloses the amino acid and nucleotide sequence of preproLHRH and its cDNA as isolated from human placental tissue. Numbers for amino acids appear within the sequence, whereas nucleotide numbers are given on the right hand side. The nucleotide sequence is that of an approximately 1500 bp cDNA clone (λ LHRH-1) isolated as described in Example 2. The cDNA for a shorter cDNA starts at nucleotide 683 (see horizontal arrow). Nucleotide differences between the two cDNAs (positions 702 and 997) are indicated. The overlined ATG indicates an alternative translational initiation codon. The Lys-Arg processing site for the precursor sequence is boxed and the glycine involved in carboxy-terminal amidation is underlined. A second possible processing site at the carboxy-terminus of the prescusor protein is hyphened. Arrows point to interruptions due to introns in the corresponding genomic DNA sequence. The AATAAA polyadenylation signal is underlined.

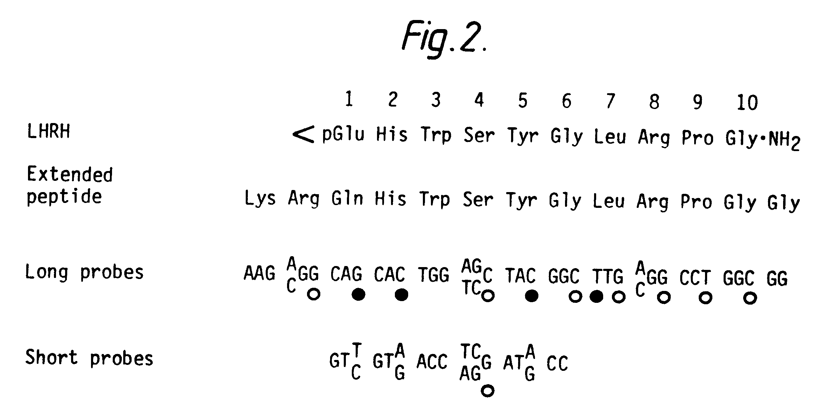

Fig. 2 discloses the chemically synthesized oligonucleotides used in the isolation of LHRH coding sequences. The amino acid sequence of LHRH (1-10) is shown together with what at the time of generating the probes was a conjectural sequence for part of the LHRH precursor ("Extended Peptide"). Based on this sequence a pool of eight oligonucleotides ("long probes", 5' to 3') was designed to be used as probes for the screening of a human genomic library. The single choice of TTG for the Leu codon was based on evolutionary arguments. The pool of sixteen heptadecamers with a sequence complementary to that coding for amino acids 1 to 6 of LHRH ("short probes", 3' to 5') was used to further select between the large number of genomic clones that hybridized with the long probes. Filled circles represent one of two, and open circles one of four possible nucleotides in third codon positions.

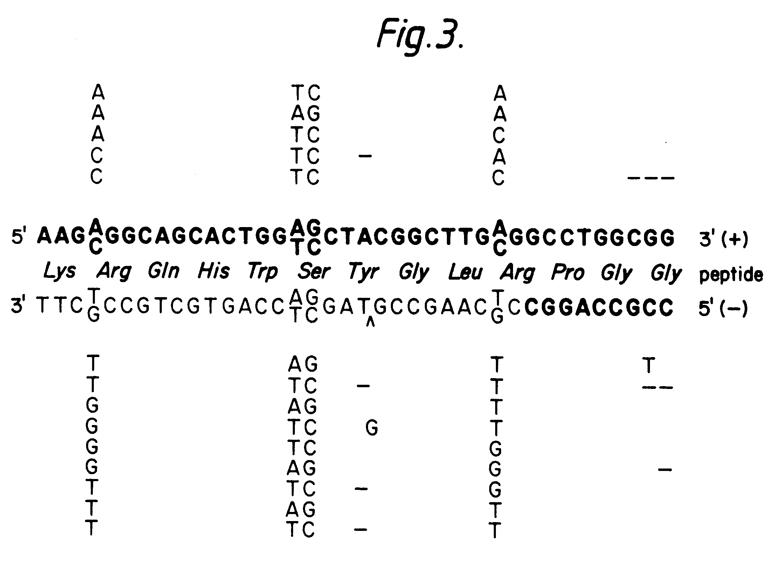

Fig. 3 illustrates the analysis of the synthesized Fig. 2 long probes. The double-stranded DNA shown is composed of the synthetic 38-mer pool (+) coding for the extended LHRH peptide (Fig. 2) and a complementary nonameric primer (both in bold print), together with the rest of the complementary strand (-) generated by enzymatic DNA synthesis (normal print). This DNA was molecularly cloned and fourteen clones were sequence-analyzed. Only nucleotides in degenerate positions are shown above and below the double-stranded DNA. Hyphens denote missing nucleotides. The position of a GMP insertion found in the Tyr codon in one of the cloned DNAs is indicated.

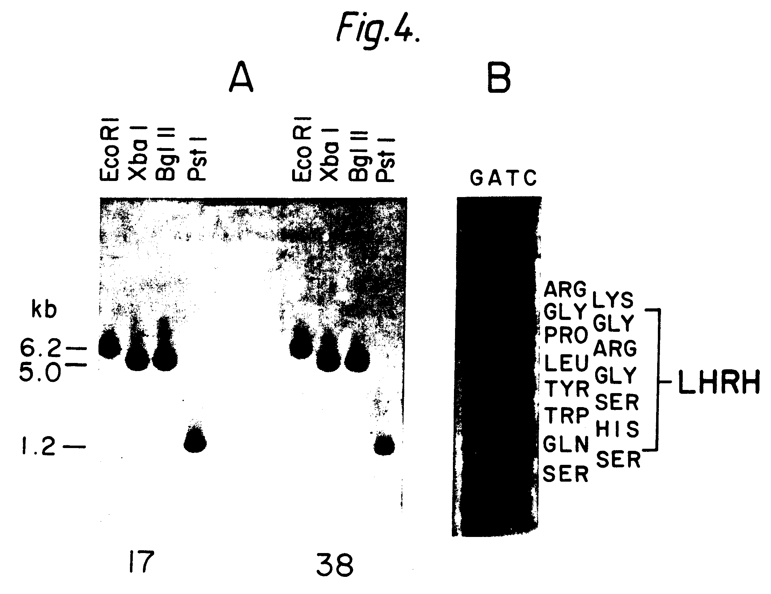

Fig. 4 illustrates the identification of a cloned human genomic DNA segment carrying LHRH coding sequences. Sequential screening of a human genomic library with two pools of oligomers [long (33) and short (17) probes; see Fig. 2] yielded one isolate, the DNA of which hybridized with both oligomeric probes. This is illustrated in panel A, which shows a Southern analysis of the DNA digested with four restriction endonucleases (EcoRI, XbaI, BglII, PstI). DNA fragment sizes in kilobase pairs (kb) are indicated on the left and were assessed using HindIII-cleaved λ DNA (Bethesda Research Laboratories). Panel B shows a sequencing gel (G,A,T,C) across the region of hybridization with both oligomer pools. The amino acid sequence encoded by the nucleotide sequence in this region is indicated.

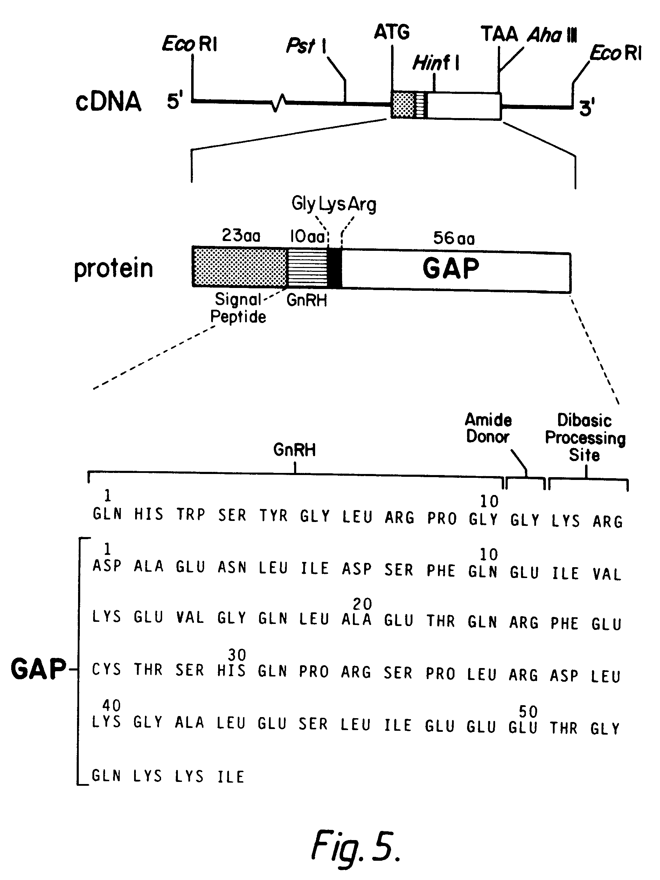

Fig. 5 depicts the restriction enzyme map, structural features and amino acid sequence for the 1500 bp cDNA isolate of Fig. 1. Note that GAP represents CTP-lys-lys-Ile.

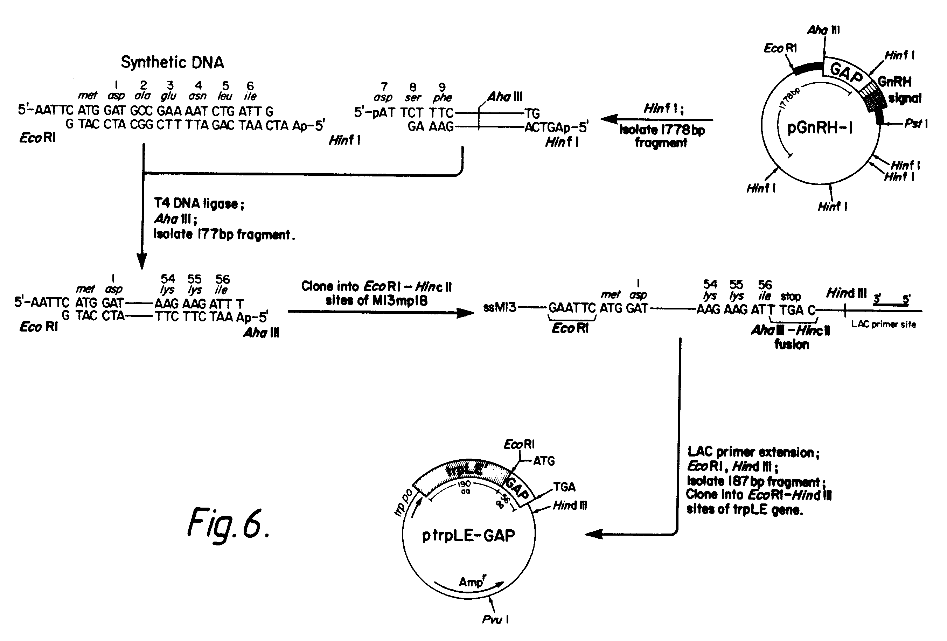

Fig. 6 discloses the preparation of an expression vector for GAP.

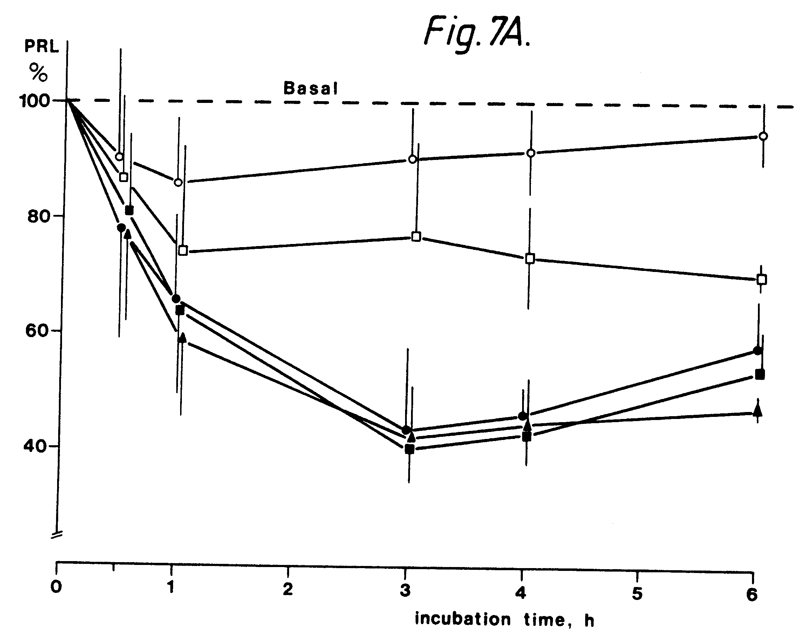

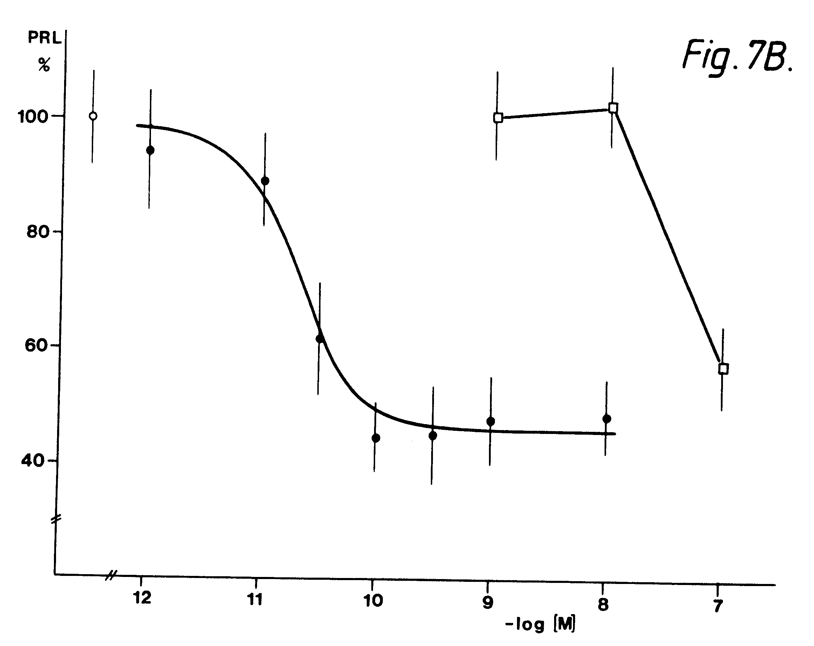

Figs. 7A and B depict the PRL inhibiting activity of GAP.

### Detailed Description

CTP is defined herein to include not only the putative 53 amino acid native sequence set forth in Fig. 1 but, for convenience, CTP is also defined to include polypeptides which are CTP fragments or mutations of CTP or such fragments. Polypeptides falling within the scope herein will be immunologically cross-reactive with CTP or will exhibit substantially the same biological activity as CTP. "Substantially the same biological activity" means that the candidate protein retains some but not necessarily all of the CTP biological functions, for example, the ability of CTP to bind to cell-surface receptors.

CTP mutations include deletions, insertions or substitutions of CTP or its fragments. For example, cys at 40 may be substituted by serine in order to improve the oxidative stability of the molecule. The putative 1ys-1ys and 1ys-arg proteolysis sites at 12-13 and/or 67-68 are eliminated by mutating the encoding DNA so that histidine is expressed instead. In addition, other residues may be mutated to enhance biological activity in patients or to improve intracellular stability in microbial expression hosts. Deletion mutations of CTP are distinguished from CTP fragments in that the amino acid sequences of fragments per se are entirely homologous with the corresponding region of CTP. Whereas deletion mutants become homologous only upon the insertion of an intermediate gap.

CTP fragments are principally C or N-terminal deletions. The CTP core fragment cys-thr-thr-his-gln-pro-arg-ser-pro-leu-arg-asp-leu-lys (residues 40-53) has been synthesized by organic rather than recombinant techniques, although it also may be produced in recombinant culture by deletion mutagenesis of the CTP cDNA or by synthesis of a truncated gene. CTP is cross-reactive with rabbit antisera raised against the core fragment of CTP and is therefore useful in CTP immunoassays as further described below. It also exhibits native biological activity, albeit diminished when compared to CTP.

CTP fusions are polypeptides having amino acid sequences including an amino acid sequence that, if excised from the fusion, would meet the foregoing definition of CTP. For example, bacterial signals such as the E. coli STII enterotoxin or alkaline phosphatase leaders may be expressed as N-terminal fusions with CTP with the objective of secreting the mature hormone.

Since CTP is putatively defined in terms of a 53 amino acid polypeptide, the C-terminal lys-lys-ile species (Fig. 1) is to be considered a fusion species, but is included within the scope of the CTP definition.

The CTP described in further detail herein is human CTP. However, like proteins from other sources such as porcine and bovine are included within the definition of CTP.

The polypeptides herein can be manufactured by synthesizing the desired amino acid sequence, but this is commercially impractical at present for longer fragments, i.e., those longer than about 15 residues, or for CTP or its fusions. Preferably, CTP is synthesized in cell culture, ordinarily recombinant cell culture.

For recombinant cell culture, DNA encoding the desired polypeptide or a portion thereof is isolated from genomic or cDNA libraries, as is further described herein, or is synthesized in vitro by known techniques. In vitro synthesis is convenient for short DNA segments, i.e., at the present those having less than about 200 bp. While genomic DNA encoding CTP or its normal fusions found in vivo are isolated from any tissues of the species whose CTP is desired, it is preferred in view of the DNA sequence data now made known herein to obtain mRMA from cells known to secrete LHRH, e.g. placenta or hypothalamus, prepare cDNA by reverse transcription in accord with known procedures, and to identify transformants containing such DNA with probes prepared in light of the Fig. 1 sequence information. The synthetic or cDNA then is tailored as required to encode the desired polypeptide and ligated into cloning vectors, or vice versa. Cloning vectors which are not suitable for later use as expression vectors are used to supply DNA for insertion into expression vectors.

A vector is a replicable DNA construct. Vectors are used herein either to amplify DNA encoding CTP (cloning vectors) and/or to express DNA which encodes the polypeptides (expression vectors). An expression vector is a replicable DNA construct in which a DNA sequence encoding a CTP polypeptide is operably linked to suitable control sequences capable of effecting the expression of the polypeptide in a suitable host. Such control sequences include a transcriptional promoter, an optional operator sequence to control transcription, a sequence encoding suitable mRNA ribosomal binding sites, and sequences which control the termination of transcription and translation.

The term "operably linked" as applied herein to DNA or protein functionalities means that one of the functionalities exerts an influence on the other. For example, DNA for a presequence, also known as a secretory leader or signal, is operably linked to DNA for a polypeptide if it is expressed as a preprotein which enables the secretion of the polypeptide; a promoter is operably linked to a coding sequence if it controls the transcription of the sequence; or a ribosome binding site is operably linked to a coding sequence if it is positioned so as to permit translation. Generally, operably linked means contiguous and, in the case of secretory leaders, contiguous and in reading phase.

Examples of vectors include plasmids, viruses (including phage), and DNA fragments which ore integratable into the host genome by recombination. Once a plasmid vector has transformed a suitable host, the vector replicates and functions independently of the host genome, or may, in the case of some viruses and fragments, integrate into the genome itself. In the present specification, "vector" is generic to "plasmid", but plasmids are the most commonly used form of vector at present. However, all other forms of vectors which serve an equivalent function and which are, or become, known in the art are suitable for use herein.

Suitable vectors will contain replicon and control sequences which are derived from species compatible with the intended host cell which is to be transfected and transformed.

Generally, a vector is selected for a given host cell so that it will contain an origin of replication, a promoter and a phenotypic selection gene, for example a gene encoding proteins conferring antibiotic resistance or supplying an auxotrophic requirement, all of which are recognized by and function in the intended host. E. coli is typically transformed using pBR322, a plasmid derived from an E. coli species [Bolivar et al., "Gene" 2: 95 (1977)]. pBR322 contains genes for ampicillin and tetracycline resistance and thus provides a convenient means for identifying transformed cells. The well-known and commercially available M13 or λ phage vectors are employed to advantage as cloning vectors.

Vectors will contain a promoter operably linked to the CTP DNA which is recognized by the host organism. Promoters most commonly used in recombinant DNA construction in prokaryotes include the β-lactamase (penicillinase) and lactose promoter systems [Chang et al., "Nature", 275: 615 (1978); Itakura et al., 198: 1056 (1977) and Goeddel et al., "Nature 281: 544 (1979)] and a tryptophan (trp) promoter system (Goeddel et al., "Nucleic Acids Res." 8: 4057 (1980) and EPO App. Publ. No. 36,776). While these are the most commonly used, other known microbial promoters are suitable. Details concerning their nucleotide sequences have been published, enabling a skilled worker operably to ligate them to DNA encoding CTP in plasmid vectors [Siebenlist et al., "Cell" 20: 269 (1980)]. The preferred vector herein is a pBR322 derivative containing the trp promoter with the Shine-Dalgarno sequence of the trp leader and a portion of the trp LE coding sequence.

Suitable host cells are prokaryotes, yeast or higher eukaryotic cells. Prokaryotes include gram negative or gram positive organisms, for example E. coli or Bacillus species. Higher eukaryotic cells include established cell lines of mammalian origin as described below. The preferred host cell is E. coli ATCC 31446, although other prokaryotes such as E. coli β, E. coli X1776 (ATCC 31,537), E. coli W3110, pseudomonas species, or Serratia Marcesans are suitable.

Transformed host cells are cells which have been transfected with a vector bearing the DNA whose expression is desired and which thereafter express protein from the DNA. Here, the expressed CTP or CTP fusion is located in the host cell cytoplasm or is secreted into either the host's periplasmic space or the culture supernatant, depending upon the host cell and signal which is employed with the CTP-encoding DNA.

Eukaryotic microorganisms, such as yeast cultures, also are transformed with CTP-encoding vectors in order to express the polypeptides herein. Saccharomyces cerevisiae, or common baker's yeast is the most commonly used among eukaryotic host microorganisms, although a number of other strains are commonly available. The plasmid YRp7 is suitable for CTP expression in yeast [Stinchcomb et al., "Nature", 282: 39 (1979); Kingsman et al., "Gene", 7: 141 (1979); Tschemper et al., "Gene", 10: 157 (1980)]. This plasmid already contains the trp1 gene which provides a selection marker for a mutant strain of yeast lacking the ability to grow in tryptophan, for example ATCC No. 44076 or PEP4-1 [Jones, "Genetics", 85: 12 (1977)]. The presence of the trp1 lesion as a characteristic of the yeast host cell genome then provides an effective environment for detecting transformants by their ability to grow in the absence of tryptophan.

Suitable promoting sequences for use in yeast vectors include the promoters for metallothionein, 3-phosphoglycerate kinase [Hitzeman et al., "J. Biol. Chem.", 255: 2073 (1980)] or other glycolytic enzymes [Hess et al., "J. Adv. Enzyme Reg.", 7: 149 (1968); and Holland et al., "Biochemistry", 17: 4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are further described in R. Hitzeman et al., EPO Publn. No. 73,657.

Other yeast promoters which are transcriptionally activated or expressed by culture growth conditions are the promoter regions for alcohol dehydrogenase 2, isocytochrome C, acid phosphatase, degradative enzymes associated with nitrogen metabolism, and the aforementioned metallothionein and glyceraldehyde-3-phosphate dehydrogenase, as well as enzymes responsible for maltose and galactose utilization. Any plasmid vector containing a yeast-compatible promoter, origin of replication and termination sequence is suitable. In constructing suitable yeast expression plasmids, the termination sequences associated with the promoted genes are ligated into the expression vector at a point 3' from the polypeptide coding sequences to provide transcriptional termination followed by polyadenylation of the mRNA.

Cultures of cells derived from multicellular organisms may also be used as hosts. This, however, is not required for CTP expression because CTP is a relatively small protein and does not require extensive processing. However, tissue cell culture is advantageous because such cells are more likely to be compatible with soluble CTP than are bacteria. In principal, any such cell culture is workable, whether from vertebrate or invertebrate culture. However, interest has been greatest in vertebrate cells, and propagation of vertebrate cells in culture (tissue culture) has become a routine procedure in recent years [Tissue Culture, Academic Press, Kruse and Patterson, editors (1973)]. Examples of such useful host cell lines are VERO and HeLa cells, Chinese hamster ovary (CHO) cell lines, and WI38, BHK, COS-7 and MDCK cell lines. Expression vectors for such cells ordinarily include (if necessary) an origin of replication, a promoter located upstream from the gene to be expressed, along with a ribosome binding site, RNA splice site (if intron-containing genomic DNA is used), a polyadenylation site, and a transcriptional termination sequence.

The transcriptional and translational control sequences in expression vectors to be used in transforming vertebrate cells are often provided by viral sources. For example, commonly used promoters are derived from polyoma, Adenovirus 2, and most preferably Simian Virus 40 (SV40). The early and late promoters are particularly useful because both are obtained easily from the virus as a fragment which also contains the SV40 viral origin of replication [Fiers et al., "Nature", 273: 113 (1978)]. Smaller or larger SV40 fragments may also be used, provided the approximately 250 bp sequence extending from the Hind III site toward the Bg1 I site located in the viral origin of replication is included. Further, it is also possible, and often desirable, to utilize human genomic promoter or control sequences normally associated with preproLHRH, provided such control sequences are compatible with the host cell systems.

An origin of replication may be provided either by construction of the vector to include an exogenous origin, such as may be derived from SV40 or other viral (e.g. Polyoma, Adenovirus, VSV, or BPV) source, or may be provided by the host cell chromosomal replication mechanism. If the vector is integrated into the host cell chromosome, the latter is often sufficient.

In selecting a preferred host mammalian cell for transfection by vectors which comprise DNA sequences encoding both a CTP and dihydrofolate reductase (DHFR), it is appropriate to select the host according to the type of DHFR protein employed. If wild type DHFR protein is employed, it ii preferable to select a host cell which is deficient in DHFR thus permitting the use of the DHFR coding sequence as a marker for successful transfection in selective medium which lacks hypoxanthine, glycine, and thymidine. An appropriate host cell in this case is the Chinese hamster ovary (CHO) cell line deficient in DHFR activity, prepared and propagated as described by Urlaub and Chasin, "Proc. Natl. Acad. Sci." (USA) 77: 4216 (1980).

On the other hand, if DHFR protein with low binding affinity for methotrexate (MTX) is used as the controlling sequence, it is not necessary to use DHFR resistant cells. Because the mutant DHFR is resistant to MTX, MTX containing media can be used as a means of selection provided that the host cells are themselves MTX sensitive. Most eukaryotic cells which are capable of absorbing MTX appear to be methotrexate sensitive. One such useful cell line is a CHO line, CHO-K1 (ATCC No. CCL 61).

In the preferred embodiment described in the Examples, a vector encoding a fusion of CTP with a bacterial protein (trp LE) is transfected into E. coli and the organism cultured. This fusion is not soluble in the cellular cytoplasm and accordingly is deposited as a refractile body within the cells. A refractile body is an insoluble aggregate of the heterologous protein, usually visible under a phase contrast microscope at magnifications as low as 1000 fold. Refractile bodies and methods for their recovery are described in EPO published application No. 114,506, published July 1, 1984. This EPO application also describes techniques for solubilizing the desired polypeptides found in refractile bodies which may be employed with CTP.

The lack of an internal methlonine residue in the CTP coding sequence facilitates the use of cyanogen bromide to cleave mature, CTP away from the bacterial portion of the fusion. However, other known methods for recovering the mature protein from a fusion, e.g. enzymatic digestions, may be employed for this purpose.

The CTP or CTP fusion may be synthesized in yeast, generally as described above, without refractile body formation. Similarly, a CTP fusion linking a selected bacterial signal sequence directly to the n-terminus of CTP can be secreted as mature, CTP, again without formation of refractile bodies.

CTP or CTP fusions are recovered from the cytoplasm, periplasm or culture supernatant of the host cell by methods known per se. The recovery process used will depend upon the nature and location of the CTP or its fusion. Periplasmic protein is recovered by osmotic shock, cytoplasmic protein by cell lysis. Then the protein is separated from other insoluble cellular matter by centrifugation and subsequently purified to the desired degree by procedures which are conventional per se, e.g., ion exchange chromatography, gel filtration, high pressure liquid chromatography, immunoaffinity column purification (using absorption to and acid desorption from immobilized anti-CTP), salting-in or salting-out precipitations, and the like.

The recovered and purified protein then is placed into dosage forms for therapeutic administration or label led with detectable markers for use in immunoassays. In addition, purified or unpurified CTP or its fragments may be employed as immunogens to raise antibodies to CTP (anti-CTP). Anti-CTP may be immobilized, e.g. by covalent bonding to an insoluble matrix or by absorption to a polyolefin surface, for use in the immunoaffinity purification of CTP or its fusions. Or it may be labelled, e.g. with an enzyme such as horseradish peroxidase or with radioiodine, for use in a sandwich-type immunoassay for the determination of CTP or its fusions. Also, CTP which is free of GnRH epitopes is useful in making immunogens for raising antibodies against predetermined CTP domains. This is enabled by the knowledge, made possible by the disclosures herein, of the amino acid sequence for CTP, and is accomplished by linking CTP fragments to immunogenic substances. This enables highly sensitive sandwich immunoassays for CTP in body fluids since antibodies can be raised against epitopes sufficiently separated that binding of antibody to one CTP epitope will not sterically inhibit the binding of labelled antibody directed against the second epitope.

CTP or its fusions are prepared for therapeutic administration by mixing the protein having the desired degree of purity with physiologically acceptable carrier, i.e., carriers which are nontoxic to recipients at the dosages and concentrations employed. Ordinarily, this will entail combining the CTP or its fusions with buffers, antioxidants, low molecular weight (less than about 10 residues) polypeptides, innocuous proteins, amino acids, carbohydrates including glucose or dextrins, and other stabilizers and excipients. This generally is accomplished in aqueous solution, after which the mixture is passed through a filter having pores sufficiently small to exclude bacteria. The sterile filtrate is filled into vials and desirably lyophilized for long-term storage.

CTP compositions are administered to animals having reproductive dysfunction or for purposes of modulating reproductive cycles, e.g. menstruation. The route of administration is in accord with known methods, e.g. intravenous, intranasal, intraperitoneal, or intramuscular infusion or injection of sterile solutions, or by timed release systems as noted below.

CTP compositions desirably are administered from an implantable timed-release article. Examples of suitable systems for proteins having the molecular weight of CTP include copolymers of L-glutamic acid and gamma ethyl-L-glutamate [u. Sidman et al., "Biopolymers" 22 (1): 547-556 (1983)], poly (2-hydroxyethyl-methacrylate) [R. Langer et al., "J. Biomed. Mater. Res." 15: 167-277 (1981) and R. Langer, "Chem. Tech." 12: 98-105 (1982)] or ethylene vinyl acetate (R. Langer et al., Id.) Such articles are implanted subcutaneously. Alternatively, CTP is embedded in an adhesive skin patch for slow transcutaneous administration.

The amount of CTP or CTP fusion that is administered will depend, for example, upon the route of administration and the condition of the patient. It will be necessary for the therapist to titer the dosage and modify the route of administration as required to obtain optimal biological activity.

The diagnostic methods used in assaying CTP, its fusions and antibodies thereto are conventional. These include the competitive, sandwich and steric inhibition techniques. The first two methods employ a phase separation step as an integral part of the method while steric inhibition assays are conducted in a single reaction mixture. The methodology for assay of CTP or its fusions on the one hand and for substances that bind CTP or its fusions on the other are essentially the same, although certain methods will be favored depending upon the size of the substance being assayed. Therefore the substance to be tested is referred to herein as an analyte, irrespective of its status otherwise as an antigen or antibody, and proteins which bind to the analyte are denominated binding partners, whether they be antibodies, cell surface receptors or antigens.

Analytical methods for CTP, its fusions, anti-CTP or CTP cell surface receptors all use one or more of the following reagents: Labelled analyte analogue, immobilized analyte analogue, labelled binding partner, immobilized binding partner and steric conjugates. The labelled reagents also are known as "tracers".

The label used is any detectable functionality which does not interfere with the binding of analyte and its binding partner. Numerous labels are known for use in immunoassay, examples including enzymes such as horseradish peroxidase, radioisotopes such as ¹⁴C and ¹³¹I, fluorophores such as rare earth chelates or fluorescein, spin labels and the like. Conventional methods are available to covalently bind these labels to proteins or polypeptides. Such bonding methods are suitable for use with CTP, CTP fusions, anti-CTP and CTP receptors, all of which are proteinaceous.

Immobilization of reagents is required for certain assay methods. Immobilization entails separating the binding partner from any analyte which remains free in solution. This conventionally is accomplished by either insolubilizing the binding partner or analyte analogue before the assay procedure, as by adsorption to a water insoluble matrix or surface (Bennich et al., U.S. 3,720,760) or by covalent coupling (for example using glutaraldehyde cross-linking), or by insolubilizing the partner or analogue afterward, e.g., by immunoprecipitation.

Steric conjugates are used in the steric hinderance method for homogeneous assay. These conjugates are synthesized by covalently linking a low molecular weight hapten to a small analyte so that antibody to hapten substantially is unable to bind the conjugate at the same time as anti-analyte. Under this assay procedure the analyte present in the test sample will bind anti-analyte, thereby allowing anti-hapten to bind the conjugate resulting in a change in the character of the conjugate hapten, e.g., a change in fluorescence when the hapten is a fluorophore. The CTP core fragment described above is suitable for use as the analyte component of the conjugate.

Other assay methods, known as competitive or sandwich assays, are well established and widely used in the commercial diagnostics industry.

Competitive assays rely on the ability of a labelled analogue (the "tracer") to compete with the test sample analyte for a limited number of binding sites on a common binding partner. The binding partner generally is insolubilized before or after the competition and then the tracer and analyte bound to the binding partner are separated from the unbound tracer and analyte. This separation is accomplished by decanting (where the binding partner was preinsolubilized) by centrifuging (where the binding partner was precipitated after the competitive reaction). The amount of test sample analyte is inversely proportional to the amount of bound tracer as measured by the amount of marker substance. Dose-response curves with known amounts of analyte are prepared and compared with the test results in order to quantitatively determine the amount of CTP, its fusions or anti-CTP present in the test sample. These heterologous assays are called ELISA systems when enzymes are used as the detectable markers.

Another species of competitive assay, homogeneous assay, does not require a phase separation. Here, a conjugate of an enzyme with the analyte is prepared so that when anti-analyte binds to the analyte the presence of the anti-analyte modifies the enzyme activity. In this case, CTP or its immunologically active fragments are conjugated with a bifunctional organic bridge to an enzyme such as peroxidase. Conjugates are selected for use with anti-CTP so that binding of the anti-CTP inhibits or potentiates enzyme activity. This method per se is widely practiced under the name EMIT.

Sandwich assays particularly are useful for the determination of CTP fusions, anti-CTP or CTP cell surface receptors, i.e., large molecules. In sequential sandwich assays an immobilized binding partner is used to adsorb test sample analyte, the test sample is removed as by washing, the bound analyte is used to adsorb labelled binding partner and bound material then separated from residual tracer. The amount of bound tracer is directly proportional to test sample analyte. In "simultaneous" sandwich assays test sample is not separated before adding the labelled binding partner. A sequential sandwich assay using anti-LHRH as one antibody and anti-CTP as the other is useful in testing samples in such a way as to distinguish proLHRH from CTP and LHRH. For example, an excess of anti-LHRH is immobilized on the inner surface of a test tube or microtiter well. This will bind both LHRH and proLHRH from the sample. Then anti-CTP is added. Anti-CTP will be bound in direct proportion to proLHRH because it does not recognize LHRH, or if it does so it will do so with greatly reduced affinity.

The foregoing are merely exemplary assays for CTP, CTP fusions, anti-CTP and CTP cell surface receptors. Other methods now or hereafter developed for the determination of these analytes are included within the scope hereof.

In order to simplify the Examples that follow, certain frequently recurring methods known to those skilled in the art are generally described below; these methods will not be described at each occurrence in the Examples other than by use of the noted key phrases.

Plasmids are designated by a low case p preceded and/or followed by capital letters and/or numbers. The starting plasmids herein are commercially available, are publically available on an unrestricted basis, or can be constructed from such available plasmids in accord with published procedures In addition, other equivalent plasmids ae known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. The various restriction enzymes used herein are commercially available and their reaction conditions, cofactors and other requirements as established by the enzyme suppliers were used. Restriction enzymes commonly are designated by abbreviations composed of a capital letter followed by other letters and then, generally, a number representing the microorganism from which each restriction enzyme originally was obtained. In general, about 1 µg of plasmid or DNA fragment is used with about 1 unit of enzyme in about 20 µl of buffer solution. Appropriate buffers and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme may be followed with bacterial alkaline phosphatase hydrolysis of the terminal 5' phosphates (dephosphorylation) to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional [T. Maniatis et al., Molecular Cloning, pp. 133-134 (1982)].

"Recovery" or "isolation" of a given fragment of DNA from a restriction digest means separation of the digest on polyacrylamide gel electrophoresis, identification of the fragment of interest by comparison of its mobility versus that of marker DNA fragments of known molecular weight, removal of the gel section containing the desired fragment, and separating the gel from DNA. This procedure is known generally. For example, see R. Lawn et al., "Nucleic Acids Res." 9: 6103-6114 (1981), and D. Goeddel et al., "Nucleic Acids Res." 8: 4057 (1980).

"Southern Analysis" is a method by which the presence of DNA sequences in a digest or DNA-containing composition is confirmed by hybridization to a known, labelled oligonucleotide or DNA fragment. For the purposes herein, unless otherwise provided, Southern analysis shall mean separation of digests on 1 percent agarose, denaturation and transfer to nltrocellulose by the method of E. Southern, "J. Mol. Biol." 98: 503-517 (1975), and hybridization as described by T. Maniatis et al., "Cell" 15: 687-701 (1978).

"Transformation" means introducing DNA Into an organism so that the DNA is replicable, either as an extrachromosomal element or chromosomal integrant. Unless otherwise provided, the method used herein for transformation of E. coli is the CaCl₂ method of Mandel et al., "J. Mol. Biol." 53: 154 (1970).

"Ligation" refers to the process of forming phosphodiester bonds between two double stranded nucleic acid fragments (T. Maniatis et al., Id., p. 146) . Unless othrwise provided, ligation may be accomplished using known buffers and conditions with 10 units of T4 DNA ligase ("ligase") per 0.5 µg of approximately equimolar amounts of the DNA fragments to be ligated.

"Preparation" of DNA from transformants means isolating plasmid DNA from microbial culture. Unless otherwise provided, the alkaline/SDS method of Maniatis et al., Id. p. 90., may be used.

"Oligonucleotides" are short length single or double stranded polydeoxynucletides which are chemically synthesized by known methods and then purified on polyacrylamide gels.

All literature citations are expressly incorporated by reference.

### Example 1

### Isolation of Genomic Sequences Coding for LHRH

A genomic library was used in preference to cDNA libraries to initially screen for LHRH encoding DNA sequences. This choice was dictated by a number of reasons, among which the anticipated low abundance of LHRH mRNA, the possibility of a very long 3' untranslated region, and the unknown location of the decapeptide coding unit within the mRNA figured prominently. The probe strategy employed in the genomic screen is illustrated in Fig. 2. The LHRH decapeptide was extended by three amino acid residues on the assumption that this peptide is preceded in its precusor protein by a processing site in the form of a pair of basic amino acids, that a Gln residue gives rise to the amino-terminal pyro Glu of LHRH, and a Gly residue donates its amino group during carboxy-terminal amidation. This extended peptide sequence was used to design a set of eight chemically synthesized [R. Crea et al., "Nucleic Acids Res." 8: 2331-2348 (1980)] oligonucleotides 38 bases in length ("long probes"). The degeneracy of these probes allowed for the two codon types for Arg (AGPu and CGX) and Ser (AGPy and TCX), but for only one of any two (filled in circles) or four (open circles) choices for all third codon positions. In addition, a set of sixteen heptadecamers ("short probes") which covered all but three of the possible coding sequences for the amino-terminal six amino acids of LHRH was synthesized and would be used below to further select candidates from the clones isolated by the long probes.

Although a similar approach using a single long probe has led to the successful isolation of several genes [S. Anderson et al., "Proc. Natl. Acad. Sci. USA" 80, 6838-6842 (1983); M. Jaye et al., "Nucleic Acids Res." 11, 2325-2335 (1983); A. Ullrich et al., "J. Embo" 3, 361-364 (1984)], the available amino acid sequence in these cases had comprised more than 20 residues. It was clear that success in isolating LHRH coding sequences would rest on the presence of all members of the pool of long probes and on the coincidence by which the correct nucleotide choices were incorporated into a sufficient number of the third codon positions. To analyze pool composition and sequence the oligonucleotides,the oligonucleotide mixture was converted into double-stranded structures using in vitro DNA synthesis in the presence of a chemically synthesized (Crea et al., Id.) nonameric primer molecule (see Fig. 3). 20 pmoles of 5' phosphorylated long probes were annealed with 40 pmoles of 5' phosphorylated nonameric primer in 40 µl of 20 mM Tris.HC1, pH 7.5, 100mM NaCl, 6 mM MgCl₂_{,} 0.1 mM EDTA, 100 µM each of dATP, dGTP, dCTP, dTTP, at 14°C for 15 min. DNA synthesis was initiated by the addition of 5 U E. coli DNA polymerase I (large fragment, Boehringer Mannheim) and allowed to proceed for 30 min at 14°C. Following phenol extraction and ethanol precipitation the DNA was incubated for 2 hrs at room temperature with 10 ng SmaI-cleaved M13 mp10 RF-DNA in 20 µl containing 50 mM Tris.Hc1, pH 8, 10 mM MgCl₂_{,} 0.1 mM EDTA, 10 mM β-mercaptoethanol, 0.5 mM rATP and T4 DNA ligase (New England Biolabs). The ligation mixture was used to transform competent E. coli JM103 cells. Recombinant phage was grown, single-stranded phage DNA prepared and sequenced using dideoxynucleoside triphosphates. Fig. 4 shows the results obtained from analyzing 14 isolates and demonstrates that, most importantly, all the nucleotide combinations of the long probes were represented. Thus, in spite of several deletions predominantly within the Tyr codon and a single nucleotide insertion in the same codon, the quality of the oligonucleotide mixture was considered satisfactory for screening the genomic library.

10⁶ λ phage containing human genomic DNA [R. Lawn, et al., "Cell" 15: 1157-1163 (1978)] were screened on duplicate filters using the long probes (see Fig. 2) which had been phosphorylated to a specific activity of 10⁷ cpm per pmole. Hybridization was at 37°C in the presence of 30 percent formamide using 10⁷ cpm per filter. Filters were washed in 3x SSC (1x SSC = 0.15 M NaCl, 0.015 M Na-citrate) at 37°C and exposed to X-ray film. Phage from 300 small areas corresponding to the number and location of positive signals were replated on individual plates and probed with long and short probes (Fig. 2). Short probe hybridization was at room temperature in the presence of 20 percent formamide and corresponding filters were washed at same termperature in 5x SSC. DNA from the single isolate which hybridized with both pools of oligomers was cleaved with restriction enzymes and DNA fragments characterized by Southern analysis using the two oligonucleotide pools,as probes under the conditions specified above. A 1200 base pair DNA fragment containing the hybridizing region was cloned into phage M13 mp18 RF-DNA and recombinant single-stranded DNA was sequenced using primed DNA synthesis in the presence of dideoxynucleoside triphosphates. The analysis of this clone is shown in Fig. 4. In panel A the same single restriction fragments are seen to hybridize with both sets of oligomers. A sequencing gel showing the hybridizing region is presented in panel B. The amino acid sequence derived from the nucleotide sequence shows that this DNA indeed codes for the LHRH peptide. The DNA sequence also predicted the expected carboxy-terminal Gly residue necessary for amidation followed by a Lys-Arg processing site. Although such a processing site had been expected to, but does not, precede LHRH (see Fig. 2), and although long probes also differed in five nucleotides from the actual LHRH coding region (cf. Figs 2 and 1), the sequence homology of these probes was sufficient to identify the correct gene. Because of the possible presence of introns in the gene an effort was made to isolate the corresponding cDNA for elucidating the complete structure of the LHRH precursor protein.

### Example 2

### Isolation of prepro LHRH cDNA

Polyadenylated RNA (10 µg) from human placental tissue was converted into double-stranded cDNA following standard procesures [T. Maniatis, et al., "Molecular Cloning, Cold Spring Harbor Laboratory" (1982)]. The cDNA was cloned using phage λgt10 as a vector according to published methods [T. Huynh, et al., "Practical Approaches in Biochemistry" (1984)]. 1.5x10⁶ recombinant phage were obtained and screened using a 600 bp Sau3A digestion fragment isolated from the genomic clone and labelled [J. Taylor, et al., "Biochem. Biophys. Acta" 4: 324-330 (1976)] to a specific activity of 10⁸ cpm per µg. Two hybridizing λ clones were isolated and found to contain cDNA inserts of approximately 1500 and 800 base pairs, respectively. These cloned cDNAs were sequenced using single-stranded recombinant M13 DNA as templates for the primed DNA synthesis in the presence of dideoxynucleoside triphosphates. Fig. 1 shows the complete nucleotide sequences of both cDNAs, together with the deduced amino acid sequence of the LHRH precursor protein (preproLHRH).

The most prominent feature of the cDNA is a very long 5'-untranslated region in excess of 1,000 nucleotides. A similarly sized 5' region has been described for the human preproenkephalin B gene but seems otherwise a rare phenomenon. since LHRH is expressed in various tissues, transcription of the precursor gene from different tissue-specific promoters might be invoked to account for the presence of such an extended 5' region in the placental cDNA. The trivial explanation that the clone is derived from an unspliced nuclear RNA can be refuted since two introns present in the gene had been clearly removed. The smaller of the two cloned cDNAs still features approximately 400 nucleotides of 5'-untranslated sequence which also happen to contain the best consensus splice acceptor site (nucleotides 960-974), thus rendering unlikely the possibility of a partially spliced version. The very low abundance of the corresponding mRNA in placental tissue has so far precluded the use of Northern analysis [H. Lehrach, et al., "Biochemistry (Wash.)" 16: 4743-4751 (1977)] to establish the correct length of its 5' region. Finally, complete colinearity of this region with the established genomic sequence rules out artifacts which might have been introduced during reverse transcription and molecular cloning.

The largest open reading frame (nucleotides 1063-1350) specifies the coding sequence for LHRH and translates into a protein of approximately 10,000 daltons which bears the hallmarks of a precursor for a polyfunctional protein. This reading frame is terminated by an ochre stop codon and followed by 160 nucleotides of 3-untranslated region containing an AATAAA sequence for polyadenylation shortly upstream of the polyA tail. The actual start of translation within the open reading frame is unclear. The first ATG initiation codon (position 1063) is followed by an in-frame ATG four codons downstream. This second ATG, but not the first, is followed by a purine and preceded by an A at position -3, as is the case with most eukaryotic initiation codons. It is concluded that this ATG at position 1075 initiates the synthesis of the LHRH precursor protein even though it involves a violation of the rule which specifies that the first ATG should bear this function [M. Kozak, "Nucleic Acids Res." 9: 5233-5252 (1981)]. It is possible that both ATGs are involved in the initiation of protein synthesis, resulting in a low translational efficiency.

The first 23 amino acids form a typical signal sequence containing a hydrophobic middle section. The putative signal sequence ends in two serine residues followed by the expected decapeptide sequence for LHRH. Cleavage of the signal peptide results in an amino-terminal glutamine residue which spontaneously or enzymatically undergoes cyclization to pyro Glu, the modified amino-terminus of LHRH. The decapeptide sequence is followed by a glycine, the standard donor of the amino group for carboxy-terminal amidation. A Lys-Arg cleavage site is present for enzymatic processing as found in many precursors to neuroendocrine peptides.
Thus, the isolated cDNAs code for a true precursor protein to LHRH which features a direct linkage of signal sequence to biologically active peptide reminiscent of the situation encountered in the precursor for vasopressin [H. Land, et al., "Nature" 295: 299-303 (1982)].

The remainder of the LHRH precursor consists of a peptide of 56 amino acid residues. It does not contain any sites for N-glycosylation (Asn-X-Thr/Ser) and no homologies could be detected when compared to other known hypothalamically produced precursors to neuroendocrine peptides. Interestingly, the carboxy-terminus of this peptide consists of the sequence LysLysIle, which is believed to represent an enzymatic cleavage site for further processing. The resulting peptide of 53 amino acids, CTP, (rather than GAP) is believed to be released along with LHRH.

### Example 3

### Construction of Plasmid Encoding a GAP Fusion

The cloned 1500 bp cDNA EcoRI fragment from Example 2 (λ LHRH-1) was subcloned into M13mp19. DNA from this M13 clone was used to isolate the 562 bp EcoRI-PstI fragment in the following way. The single strand M13 template was made double stranded (as required for restriction enzyme cleavage) by priming the DNA with the M13 17-mer universal sequencing primer in the presence of the four dNTP's and the Klenow fragment of DNA polymerase I. Double stranded DNA was digested with EcoRI and PstI and the 562 bp fragment was recovered by polyacrylamide gel electrophoresis and electroelution. This fragment was ligated to pUC 11 which had been digested with PstI and EcoRI, E. coli JM83 transformed with the ligation mixture and a plasmid pGnRH-1 (Fig. 6) was recovered from one of the colonies.

pGnRH-1 contains five sites recognized by HinfI. Thus, when pGnRH-1 is digested with HinfI, five fragments are produced. The largest of these, the 1778 bp HinfI fragment, was isolated and this fragment ligated to a synthetic oligonucleotide (Fig. 6) encoding the 5' end of met-GAP sequence. The oligonucleotide is synthesized with a sticky end that only binds to the HinfI sticky end (GATTC) found in the GAP coding region; the other HinfI site contains the sequence GACTC which is not self complementary to the HinfI sticky end in the synthetic DNA. This oligonucleotide was made by synthesizing the two strands, phosphorylating chain II (the lower strand in Fig. 6) with polynucleotide kinase, and annealing the two strands together. The ligation product was then digested with AhaIII (forming a blunt end) and the 177 bp AhaIII - EcoRI fragment (encoding GAP fused to the C-terminal tripeptide) recovered.

The 177 bp fragment was ligated to the M13 phage mp18 which had been digested with EcoRI and HincI (forming a blunt end and an EcoRI sticky end). E. coli JM101 was transformed with the ligation mixtures and the phage mp18GAP-1 (not shown) isolated from a white plaque. The sequence of the 177 bp fragment was confirmed on ss M13 template by dideoxy sequencing. Single-stranded mp18-GAP DNA was made double-stranded in vitro by elongating the 17-mer LAC primer (J. Messing et al., 1981, "Nucl. Acids. Res." 9: 309-321) in the presence of dNTPs and Klenow. mp18GAP-1 DNA was digested with EcoRI and Hind III, and the 187bp fragment isolated.

ptrpLE, (not shown) a modification of pNCV [Davis et al., "P.N.A.S." 78: 5376 (1981)], lacking tetracycline resistance and including ampicillin resistance and a polylinker region derived from M13 mp18, was digested with EcoRI and Hind III. The digested plasmid DNA was ligated to the 187bp EcoRI-Hind III fragment, the ligation used to transform E. coli 294 cells (ATCC 31446) and transformants identified by Southern analysis using the 562 bp PstI-EcoRI CTP fragment which had been ³²P labelled. ptrpLE-GAP was recovered from a transformant colony.

A similar vector encoding CTP was made by in vitro mutagenesis of the GAP lys 54 codon of mp18GAP-1 to a stop codon. The GAP fusion was more stable to cytoplasmic E. coli proteases than CTP.

### Example 4

### Preparation of Anti-CTP

The CTP core fragment NH₂-cys-thr-thr-his-gln-pro-arg-ser-pro-leu-arg-asp-leu-lys-COOH, was synthesized. This fragment then was covalently linked to bovine serum albumin (BSA) by crosslinking through cys40 with suberic acid bis (N-hydroxy-succinimide ester) using known techniques. Three rabbits were immunized with the BSA conjugate. All three developed polyclonal antibodies to the fragment, although in varying titers. Serum was harvested from an immunized rabbit and radioiodinated with ^{131I} using the chloramine T procedure. This labelled antiserum was used to monitor the purification of the CTP fusion expressed and purified in Example 5, but would be suitable for assay of CTP or its fusions in clinical test samples such as serum.

### Example 5

### Expression and Recovery of a GAP Fusion

E. coli 294 (ATCC 31446) cells were transformed with ptrpLE-GAP. Transformants were innoculated into 1/2 liter of M9 culture medium containing 50 mg/ml ampicillin and grown to a cell density of about 3 OD₅₅₀. Cells were separated from the stationary phase culture medium and refractile bodies recovered by lysis in 2 percent SDS and 1 percent beta-mercaptoethanol and precipitated with 10 vols. of cold acetone. High density culture used E. coli strain K12 W3110 cells. Pelleted refractile bodies were homogenized in 20 ml of 6M guanidine hydrochloride in order to solubilize the protein. The soluble proteins were dialyzed into 4M urea and applied to a Vydac C4, 300Å, 15-20 µm, 5x35 cm column (a silica hydrophobic affinity column) and separated on reverse phase HPLC at a flow rate of 15 ml/min. using a gradient of 25-60 percent acetonitrile/0.1 percent trifluoroacetic acid. 15 ml fractions were collected. Anti-CTP reactive material eluted at about 49 percent vol/vol acetonitrile.

Anti-CTP reactive fractions were lyophilized and then cleaved by cyanogen bromide (CNBr) in known manner: About 5.9 mg of protein was dissolved in 480 µl of 70 percent formic acid. 41.9 µl of CNBr solution (100mg/ml in formic acid) and 18.8 µl Na S₂ O₃ solution (10 mg/ml in formic acid) were added to the dissolved protein and the reaction mixture incubated for 24 hours while shaking. The reaction mixture was diluted into 0.1 percent TFA and chromatographed on a Vydac C18, 300Å, 15-20 µm, 2.5x35 cm column (a C18 silica hydrophobic affinity column) and eluted with a 30-50 percent acetonetrile/0.1 percent TFA gradient. The fractions containing immunoreactive material were pooled and lyophilized.

This material was dissolved in 2M acetic acid and gel filtered on a Sephadex G-50 column, eluting with 2M acetic acid. Immunoreactive material from the eluate was purified on reverse phase HPLC using a Vydac C4, 300Å, 5 µm, 1x25 cm column (a C-4 hydrophobic affinity resin) using a 33-45 percent acetonetrile/0.1 percent TPA gradient elution. The 40 percent vol/vol acetonitrile eluate contained a single protein peak and was judged to be homogeneous. The product was confirmed to be GAP by amino acid sequencing and analysis.

### Example 6

### Biological Activity of GAP

Pituitary cells from female Sprague-Dawley rats were established in cell culture by the method of K. Nikolics et al., "Peptides" 2: 65-73 (1981). 1 ml of about 1x10⁻⁸ molar concentration of GAP or LHRH in medium 199 (a nutrient medium) was added to a four day pituitary cell culture per 5x10⁵ cells. After 4 hours incubation at 37°C in 5 percent CO₂/95 percent air the cell culture supernatant fluids were assayed for release of LH and FSH. The results are shown in the Table below.

**Table 1**

| Test Sample | LH (pg)/5x10⁵ cells | FSH (pg)/5x10⁵ cells |
|---|---|---|
| Basal | 4293 ± 474 | 2701 ± 718 |
| LHRH 10⁻⁸M | 30503 ± 3342 | 11403 ± 350 |
| GAP 10⁻⁸M | 25825 ± 2655 | 10496 ± 838 |

From this data GAP is shown to be active in stimulating pituitary cells to release LH and FSH, and accordingly to be useful as an alternative to LHRH in the modulation of reproductive cycles. In a similar experiment the CTP-core polypeptide of Example 4 also was active in LH and FSH release, albeit at higher concentrations than required for GAP.

### Example 7

### Prolactin Release-Inhibiting and Gonadotropin Releasing Action of GAP

To further investigate the prolactin inhibiting and gonadotropin releasing action of GAP, concentration-respose relationships were determined and combined applications with factors known to influence the secretion of these anterior pituitary hormones were conducted.

The anterior pituitary cell culture from female Sprague-Dawley rats was prepared as described in Example 6. In Fig. 7A the indicated concentrations of GAP (open circles, 10⁻¹¹M; open squares, 3x10⁻¹¹M; closed circles 10⁻¹⁰M; closed squares, 10⁻⁹M; and triangle 10⁻⁸M) were incubated with 5x10⁵ cells/ml medium in 24-well culture plates for the indicated time. Media were assayed for PRL and values were expressed in terms of rPRL-RP-2 reference protein (National Pituitary and Hormone Program). The points are means ± standard deviation of four parallel culture wells of which duplicate samples were assayed by RIA. Basal levels were determined from eight parallel wells. The curves shown represesent data obtained in three to five independent cell cultures.

Fig. 7B depicts PRL secretion from the rat pituitary cells in the presence of increasing concentrations of GAP (closed circle) and dopamine (square). Control value without additions is the open circle. PRL secretion is expressed as a percent of uninhibited basal levels.

GAP exerted a significant inhibitory effect on the secretion of prolactin from pituitary lactotrophs. This effect was dose- and time-dependent, as shown in Fig. 7, with a maximal inhibition to 45-50 percent of the basal value. This inhibitory action of the peptide could be observed at very low concentrations. The magnitude of the inhibitory action of GAP on PRL secretion was comparable to that of dopamine (Fig. 7B). The two compounds did not act synergistically at lower concentrations. Thyrotropin releasing hormone (TRH), which stimulates the release of prolactin, caused a significant shift in the dose required for prolactin inhibition. In the presence of 10⁻⁷M TRH, higher does of GAP were required for maximal inhibition.

Increasing concentrations of GAP caused an increasing LH and FSH secretory response from rat anterior pituitary cells (Table 1). The maximum stimulation of the release of both gonadotropins was less than that induced by GnRH. Similar to GnRH, GAP stimulated both LH and FSH release. Half-maximal stimulation of LH release by GAP occurred at somewhat higher concentrations than by GnRH (2x10⁻⁹M for GAP vs. 8x10⁻¹⁰M for GnRH). The two peptides, when applied together, did not show additive or synergistic characteristics but caused the same response as obtained with GnRH alone. However, GAP stimulated FSH secretion at approximately the same concentrations as GnRH with ED₅₀ values of the order of 5x10⁻¹⁰M. The combination of GAP and GnRH did not result in significant additive activity.

It should be noted that the effective concentration range for the prolactin release inhibiting effect is lower than the corresponding gonadotropin release, suggesting that receptors which mediate the former action have a higher affinity for the peptide than receptors on gonadotrophs. Since lactotrophs are more abundant in the anterior pituitary than gonadotrophs, it is likely that the majority of the peptide molecules is normally bound to lactotrophs. Therefore, prolactin inhibition seems to be the primary action of CTP which can therefore be appropriately termed PIF. As a result, CTP species such as GAP are useful in treating diseases dependent upon prolactin and in modulating menses.

Examples of diseases dependent upon prolactin include hypogonadism in males and amenorrhea in females which are dependent upon hyperprolactinemia, as well as prolactin dependent tumors, especially mammary neoplasm. Potency in males and normal menstrual cycles in females are restored by administration of therapeutically effective doses of CTP species in pharmaceutically acceptable carriers. Suitable carriers are described above. Doses will be titered to the delivery mode and condition of the patient. However, in general a dose is sufficient which is effective to reduce plasma prolactin concentrations levels to basal levels, about from 5 to 25 ng/ml, as determined by conventional immunoassay in bromocriptive therapy (S. Sander et al., 1984, "J. Clinical Endocrinology and Metabolism" 59(5): 941-948: S. Winters et al ., 1984, "Clinical Endocrinology 21: 257-263; and A. Klibanski et al., 1984, "J. Clinical Endocrinology and Metabolism" 58(6): 1141-1147).

CTP compositions also are useful for veterinary purposes, primarily in rapidly restoring memses in postpartum animals and in modulating lactation. Lactation modulation for economically significant purposes generally means reducing the plasma levels of CTP in order to derepress PRL synthesis and release from the pituitary. This is best accomplished by immunizing dairy animals such as cattle against CTP by the administration of immunogenic CTP conjugates, e.g. the bacterial protein-CTP fusions described above or the BSA-CTP fragment conjugate described in Example 4. While it is possible to administer anti-CTP for passive immunization against CTP, for economic reasons it is preferable to actively antiimmunize the animal. Animal GTP is identified by probing genomic libraries from the species in question with a ³²p-labelled 1500bp cDNA fragment of Example 2 to identify the CTP precursor DNA for the species of interest, sequencing the animal gene, and thereafter expressing it in recombinant culture or synthesizing it (or a core fragment thereof) by in vitro methods. Alternatively, antibodies raised against human CTP which cross-react with the CTP of the immunized species are useful.

## Claims (Claims for the following Contracting State(s): BE, CH, DE, FR, GB, IT, LI, LU, NL, SE)

1. C-terminal polypeptide (CTP) having the amino acid sequence as depicted at amino acids 14 to 66 of Fig 1, or which is a mutation, excluding fusions other than with C-terminal lys lys ile, or fragment of said depicted sequence, or a mutation of a said fragment, the fragment or mutation being immunologically cross reactive with CTP as depicted at amino acids 14 to 66 of Fig 1 or having substantially the same biological activity, said CTP being free of LHRH.

2. The polypeptide according to claim 1 having the amino acid sequence depicted at amino acids 14 to 66 of Fig 1.

3. A CTP fusion, other than with LHRH the fusion including CTP having the amino acid sequence as depicted at amino acids 14 to 66 of Fig 1, or which is a mutation or fragment of said depicted sequence, or a mutation of a said fragment, the fragment or mutation being immunologically cross reactive with CTP as depicted at amino acids 14 to 66 of Fig 1 or having substantially the same biological activity.

4. An immunogenic conjugate of a CTP of claim 1 or claim 2 or of a CTP fusion according to claim 3.

5. An immunogenic conjugate according to claim 4 wherein said CTP or CTP fusion is covalently cross linked to an immunogenic protein.

6. A composition comprising: C-terminal polypeptide (CTP) of claim 1 or claim 2, a fusion of claim 3, or an immunogenic conjugate of claim 4 or claim 5, for pharmaceutical use.

7. A composition of claim 6 which comprises a semipermeable barrier through which the polypeptide will diffuse at a predetermined rate.

8. A composition of claim 6 in a vaccine.

9. The use of an immunogenic conjugate of claim 4 for producing antibodies to CTP.

10. The use according to Claim 9 for producing antibodies to CTP in a dairy animal.

11. The use of C-terminal polypeptide (CTP), the amino acid sequence of said CTP being as depicted at amino acids 14 to 66 of Fig 1 or said CTP being a mutation or fragment of said depicted sequence, or a mutation of a said fragment, the fragment or mutation being immunologically cross reactive with CTP as depicted at amino acids 14 to 66 of Fig 1 or having substantially the same biological activity; or of a fusion of said CTP; in the preparation of a medicament for inhibition of prolactin release.

12. The use according to claim 11 wherein the medicament is for treatment of hyperprolactinemia dependent hypogonadism, or amenorrhea, or of prolactin dependent tumors.

13. The use of C-terminal polypeptide (CTP) of claim 1 or claim 2, or of a fusion of claim 3 in the preparation of a medicament for release of LH and FSH.

14. Antibody capable of binding to C-terminal polypeptide (CTP) of claim 1 or claim 2.

15. Antibody according to claims 14 labelled with a detectable marker or insolubilised.

16. C-terminal polypeptide (CTP) of claim 1 or claim 2, or a CTP fusion of claim 3 labelled with a detectable marker.

17. A diagnostic method comprising obtaining a test sample and determining the amount of CTP in said sample the CTP having the amino acid sequence as depicted at amino acids 14 to 66 of Fig 1, or being a mutation, excluding fusions other than with C-terminal lys lys ile or fragment of said depicted sequence, or a mutation of a said fragment, the fragment or mutation being immunologically cross reactive with CTP as depicted at amino acids 14 to 66 of Fig 1 or having substantially the same biological activity.

18. A method for synthesizing a polypeptide of claim 1 or claim 2 or a fusion of claim 3; the method comprising:
(a) transforming a host cell with a vector known to encode the polypeptide or fusion; and
(b) culturing the cell.

19. The method of claim 18 wherein the polypeptide is CTP having the amino acid sequence depicted at amino acids 14 to 66 of Fig 1, or is a fusion of said depicted sequence with another polypeptide.

20. The method of claim 19 wherein the fusion is a microbial polypeptide bound to the N-terminus of CTP, or the tripeptide lys-lys-ile bound to the C-terminus of CTP.

21. The method of claim 20 wherein the microbial polypeptide is a bacterial signal operably linked to CTP for the secretion of CTP.

22. A DNA isolate encoding the polypeptide of any one of claims 1 or 2, the fusion of claim 3 or conjugate of claim 4, provided that said DNA does not encode a CTP fusion with LHRH.

23. The DNA of claim 22 which is operably ligated into a replicable vector.

24. A host cell transformed with the vector of claim 23.

25. Use of an antibody of claim 14 in the manufacture of a composition for passive immunisation against CTP.

## Claims (Claims for the following Contracting State(s): AT)

1. A process which comprises the preparation of C-terminal polypeptide (CTP) having the amino acid sequence as depicted at amino acids 14 to 66 of Fig 1, or which is a mutation, excluding fusions other than with C-terminal lys lys ile, or fragment of said depicted sequence, or a mutation of a said fragment, the fragment or mutation being immunologically cross reactive with CTP as depicted at amino acids 14 to 66 of Fig 1 or having substantially the same biological activity, said CTP being free of LHRH.

2. The process of claim 1 wherein the polypeptide is CTP, having the amino acid sequence depicted at amino acids 14 to 66 of Fig 1.

3. A process which comprises the preparation of a CTP fusion, other than with LHRH, the fusion including CTP having the amino acid sequence as depicted at amino acids 14 to 66 of Fig 1, or which is a mutation or fragment of said depicted sequence, or a mutation of a said fragment, the fragment or mutation being immunologically cross reactive with CTP as depicted at amino acids 14 to 66 of Fig 1 or having substantially the same biological activity.

4. A process which comprises the preparation of an immunogenic conjugate of a CTP as obtainable by the process of claim 1 or claim 2 or of a CTP fusion as obtainable by the process of claim 3.

5. A process according to claim 4 wherein said CTP or CTP fusion is covalently cross linked to an immunogenic protein.

6. The use of C-terminal polypeptide (CTP) as obtainable by the process of claim 1 or claim 2, of a fusion as obtainable by the process of claim 3, or of an immunogenic conjugate as obtainable by the process of claim 4 or claim 5 in the preparation of a pharmaceutical product.

7. The use according to claim 6 wherein the pharmaceutical product comprises a semipermeable barrier through which the polypeptide will diffuse at a predetermined rate.

8. The use according to claim 6 wherein the pharmaceutical product is a vaccine.

9. The use of an immunogenic conjugate as obtainable by the process of claim 4 for producing antibodies to CTP.

10. The use according to Claim 9 for producing antibodies to CTP in a dairy animal.

11. The use of C-terminal polypeptide, (CTP) the amino acid sequence of said CTP being as depicted at amino acids 14 to 66 of Fig 1 or said CTP being a mutation or fragment of said depicted sequence, or a mutation of a said fragment, the fragment or mutation being immunologically cross reactive with CTP as depicted at amino acids 14 to 66 of Fig 1 or having substantially the same biological activity; or of a fusion of said CTP; in the preparation of a medicament for inhibition of prolactin release.

12. The use according to claim 11 wherein the medicament is for treatment of hyperprolactinemia dependent hypogonadism, or amenorrhea, or of prolactin dependent tumours.

13. The use of C-terminal polypeptide (CTP) as obtainable by the process of claim 1 or claim 2, or of a fusion as obtainable by the process of claim 3 in the preparation of a medicament for release of LH and FSH.

14. A process which comprises the preparation of an antibody capable of binding to C-terminal polypeptide (CTP) as obtainable by the process of claim 1 or 2.

15. A process according to claim 14 further comprising labelling the antibody with a detectable marker or insolubilising it.

16. A process which comprises labelling C-terminal polypeptide (CTP) as obtainable by the process of claim 1 or claim 2 or a fusion as obtainable by the process of claim 3 wherein the polypeptide or fusion is labelled with a detectable marker.

17. A diagnostic method comprising obtaining a test sample and determining the amount of CTP in said sample the CTP having the amino acid sequence being as depicted at amino acids 14 to 66 of Fig 1, or being a mutation, excluding fusions other than with C-terminal lys lys ile or fragment of said depicted sequence, or a mutation of a said fragment, the fragment or mutation being immunologically cross reactive with CTP as depicted at amino acids 14 to 66 of Fig 1 or having substantially the same biological activity.

18. A process according to any one of claims 1 to 3 for synthesizing a polypeptide or fusion; the method comprising:
(a) transforming a host cell with a vector known to encode the polypeptide or fusion; and
(b) culturing the cell.

19. The process of claim 18 wherein the polypeptide is CTP having the amino acid sequence depicted at amino acids 14 to 66 of Fig 1, or is a fusion of said depicted sequence with another polypeptide.

20. The process of claim 19 wherein the fusion is a microbial polypeptide bound to the N-terminus of CTP, or the tripeptide lys-lys-ile bound to the C-terminus of CTP.

21. The process of claim 20 wherein the microbial polypeptide is a bacterial signal operably linked to CTP for the secretion of CTP.

22. A process which comprises the preparation of a DNA isolate encoding the polypeptide or fusion as obtainable by the process of any one of claims 1 to 3, provided that said DNA does not encode a CTP fusion with LHRH.

23. The process of claim 22 wherein said DNA is operably ligated into a replicable vector.

24. A host cell transformed with the vector of claim 23.

25. Use of an antibody as obtainable by the process of claim 14 in the manufacture of a composition for passive immunisation against CTP.

## Patentansprüche (Patentansprüche für folgende(n) Vertragsstaat(en): BE, CH, DE, FR, GB, IT, LI, LU, NL, SE)

1. C-terminales Polypeptid (CTP), das die Aminosäuresequenz aufweist, die bei Aminosäuren 14 bis 66 in Fig.1 dargestellt ist, oder die eine Mutation, mit Ausnahme anderer Fusionen als mit C-terminalem lys-lys-ile, oder ein Fragment genannter dargestellter Sequenz, oder eine Mutation eines genannten Fragments ist, wobei das Fragment oder die Mutation mit CTP, wie bei Aminosäuren 14 bis 66 in Fig.1 dargestellt, immunologisch kreuzreaktiv sind oder im wesentlichen die gleiche biologische Aktivität aufweisen, wobei genanntes CTP LHRH-frei ist.

2. Polypeptid nach Anspruch 1 mit der Aminosäuresequenz, die bei Aminosäuren 14 bis 66 in Fig.1 dargestellt ist.

3. CTP Fusion, die eine andere als mit LHRH ist, wobei die Fusion CTP enthält, das die Aminosäuresequenz, wie sie bei Aminosäuren 14 bis 66 in Fig.1 dargestellt ist, besitzt, oder die eine Mutation oder ein Fragment genannter dargestellter Sequenz oder eine Mutation eines genannten Fragments ist, wobei das Fragment oder die Mutation mit CTP, wie bei Aminosäuren 14 bis 66 in Fig. 1 dargestellt, immunologisch kreuzreaktiv sind oder im wesentlichen die gleiche biologische Aktivität aufweisen.

4. Immunogenisches Konjugat eines CTP nach Anspruch 1 oder 2 oder einer CTP-Fusion nach Anspruch 3.

5. Immunogenisches Konjugat nach Anspruch 4, worin genanntes CTP oder genannte CTP Fusion kovalent mit einem immunogenischen Protein vernetzt ist.

6. Zusammensetzung, enthaltend C-terminales Polypeptid (CTP) nach Anspruch 1 oder 2, eine Fusion nach Anspruch 3 oder ein immunogenisches Konjugat nach Anspruch 4 oder 5, zur pharmazeutischen Verwendung.

7. Zusammensetzung nach Anspruch 6, die eine halbdurchlässige Barriere enthält, durch die das Polypeptid mit einer festgelegten Geschwindigkeit diffundiert.

8. Zusammensetzung nach Anspruch 6 in einem Impfstoff.

9. Verwendung eines immunogenischen Konjugates nach Anspruch 4 zur Herstellung von Antikörpern gegen CTP.

10. Verwendung nach Anspruch 9 zur Herstellung von Antikörpern gegen CTP in Milchvieh.

11. Verwendung eines C-terminalen Polypeptids (CTP), wobei die Aminosäuresequenz des genannten CTP so beschaffen ist wie sie bei Aminosäuren 14 bis 66 in Fig.1 dargestellt ist, oder wobei genanntes CTP eine Mutation oder ein Fragment genannter dargestellter Sequenz oder eine Mutation eines genannten Fragments ist; wobei das Fragment oder die Mutation mit CTP, wie es bei Aminosäuren 14 bis 66 in Fig.1 dargestellt ist, immunologisch kreuzreaktiv sind oder im wesentlichen die gleiche biologische Aktivität aufweisen; oder einer Fusion des genannten CTP, bei der Herstellung eines Medikaments zur Hemmung der Prolactin-Freisetzung.

12. Verwendung nach Anspruch 11, worin das Medikament der Behandlung von Hyperprolactinämie-abhängigen Hypogonadismus, Amenorrhoe oder Prolactin-abhängigen Tumoren dient.

13. Verwendung eines C-terminalen Polypeptids (CTP) nach Anspruch 1 oder 2, oder einer Fusion nach Anspruch 3, bei der Herstellung eines Medikaments zur Freisetzung von LH und FSH.

14. Antikörper, der zur Bindung an das C-terminale Polypeptid (CTP) nach Anspruch 1 oder 2 fähig ist.

15. Antikörper nach Anspruch 14, der mit einem nachweisbaren Marker markiert oder insolubilisiert ist.

16. C-terminales Polypeptid (CTP) nach Anspruch 1 oder 2, oder eine CTP-Fusion nach Anspruch 3, das/die mit einem nachweisbaren Marker markiert ist.

17. Diagnostisches Verfahren, das die Entnahme einer Testprobe und die Bestimmung der CTP-Menge in genannter Probe umfaßt, wobei das CTP jene Aminosäuresequenz hat, die bei den Aminosäuren 14 bis 66 in Fig.1 dargestellt ist, oder eine Mutation mit Ausnahme anderer Fusionen als mit C-terminalem lys-lys-ile, oder Fragment genannter dargestellter Sequenz, oder eine Mutation eines genannten Fragments ist, wobei das Fragment oder die Mutation mit CTP, wie bei den Aminosäuren 14 bis 66 in Fig.1 dargestellt, immunologisch kreuzreaktiv sind oder im wesentlichen die gleiche biologische Aktivität aufweisen.

18. Verfahren zur synthetischen Herstellung eines Polypeptids nach Anspruch 1 oder 2, oder einer Fusion nach Anspruch 3, wobei das Verfahren umfaßt:
(a) Transformieren einer Wirtszelle mit einem Vektor, von dem bekannt ist, daß er für das Polypeptid oder die Fusion kodiert; und
(b) Kultivierung der Zelle.

19. Verfahren nach Anspruch 18, worin das Polypeptid CTP ist, das jene Aminosäuresequenz hat, die bei den Aminosäuren 14 bis 66 in Fig.1 dargestellt ist, oder eine Fusion genannter dargestellter Sequenz mit einem anderen Polypeptid ist.

20. Verfahren nach Anspruch 19, worin die Fusion ein mikrobielles, an den N-Terminus von CTP gebundenes Polypeptid oder das an den C-Terminus von CTP gebundene Tripeptid lys-lys-ile ist.

21. Verfahren nach Anspruch 20, worin das mikrobielle Polypeptid ein bakterielles Signal ist, das für die Sekretion von CTP operabel mit CTP verbunden ist.

22. DNA-Isolat, das für das Polypeptid nach einem der Ansprüche 1 oder 2, die Fusion nach Anspruch 3, oder das Konjugat nach Anspruch 4 kodiert, mit der Maßgabe, daß genannte DNA nicht für eine CTP-Fusion mit LHRH kodiert.

23. DNA nach Anspruch 22, die operabel in einen replizierbaren Vektor ligiert ist.

24. Wirtszelle, die mit dem Vektor nach Anspruch 23 transformiert ist.

25. Verwendung eines Antikörpers nach Anspruch 14 bei der Herstellung einer Zusammensetzung zur passiven Immunisierung gegen CTP.

## Patentansprüche (Patentansprüche für folgende(n) Vertragsstaat(en): AT)

1. Verfahren, das die Herstellung von C-terminalem Polypeptid (CTP) umfaßt, das jene Aminosäuresequenz besitzt, die bei Aminosäuren 14 bis 66 in Fig.1 dargestellt ist, oder die eine Mutation mit Ausnahme anderer Fusionen als mit C-terminalem lys-lys-ile, oder Fragment genannter dargestellter Sequenz oder eine Mutation eines genannten Fragments ist, wobei das Fragment oder die Mutation mit CTP, wie bei Aminosäuren 14 bis 66 in Fig.1 dargestellt, immunologisch kreuzreaktiv sind oder im wesentlichen die gleiche biologische Aktivität haben, wobei genanntes CTP LHRH-frei ist.

2. Verfahren nach Anspruch 1, worin das Polypeptid CTP ist, das jene Aminosäuresequenz besitzt, die bei Aminosäuren 14 bis 66 in Fig.1 dargestellt ist.

3. Verfahren, das die Herstellung einer anderen CTP-Fusion als mit LHRH umfaßt, wobei die Fusion CTP enthält, das jene Aminosäuresequenz besitzt, die bei Aminosäuren 14 bis 66 in Fig.1 dargestellt ist, oder das/die eine Mutation oder ein Fragment genannter dargestellter Sequenz oder eine Mutation eines genannten Fragments ist, wobei das Fragment oder die Mutation mit CTP, wie bei Aminosäuren 14 bis 66 in Fig.1 dargestellt, immunologisch kreuzreaktiv sind oder im wesentlichen die gleiche biologische Aktivität haben.

4. Verfahren, das die Herstellung eines immunogenischen Konjugats von einem CTP, wie es durch das Verfahren nach Anspruch 1 oder Anspruch 2 gewonnen werden kann, oder einer CTP-Fusion, wie sie durch das Verfahren nach Anspruch 3 gewonnen werden kann, umfaßt.

5. Verfahren nach Anspruch 4, worin genanntes CTP oder genannte CTP-Fusion kovalent mit einem immunogenischen Protein vernetzt sind.

6. Verwendung von C-terminalem Polypeptid (CTP), wie es durch das Verfahren nach Anspruch 1 oder 2 gewonnen werden kann, einer Fusion, wie sie durch das Verfahren nach Anspruch 3 gewonnen werden kann, oder eines immunogenischen Konjugats, wie es durch das Verfahren nach Anspruch 4 oder 5 gewonnen werden kann, bei der Herstellung eines pharmazeutischen Produktes.

7. Verwendung nach Anspruch 6, worin das pharmazeutische Produkt eine halbdurchlässige Barriere umfaßt, durch die das Polypeptid mit festgelegter Geschwindigkeit diffundiert.

8. Verwendung nach Anspruch 6, worin das pharmazeutische Produkt ein Impfstoff ist.

9. Verwendung eines immunogenischen Konjugates, wie es durch das Verfahren nach Anspruch 4 gewonnen werden kann, zur Herstellung von Antikörpern gegen CTP.

10. Verwendung nach Anspruch 9 zur Herstellung von Antikörpern gegen CTP in Milchvieh.

11. Verwendung von C-terminalem Polypeptid (CTP), wobei die Aminosäuresequenz des genannten CTP so beschaffen ist, wie sie bei den Aminosäuren 14 bis 66 in Fig.1 dargestellt ist, oder wobei genanntes CTP eine Mutation oder ein Fragment genannter dargestellter Sequenz oder eine Mutation eines genannten Fragments ist, wobei das Fragment oder die Mutation mit CTP, wie bei Aminosäuren 14 bis 66 in Fig.1 dargestellt, immunologisch kreuzreaktiv sind oder im wesentlichen die gleiche biologische Aktivität haben; oder einer Fusion von genanntem CTP bei der Herstellung eines Medikaments zur Hemmung der Prolactin-Freisetzung.

12. Verwendung nach Anspruch 11, worin das Medikament der Behandlung von Hyperprolactinämie-abhängigem Hypogonadismus, Amenorrhoe oder Prolactin-abhängigen Tumoren dient.

13. Verwendung von C-terminalem Polypeptid (CTP), wie es durch das Verfahren nach Anspruch 1 oder Anspruch 2 gewonnen werden kann, oder einer Fusion, wie sie durch das Verfahren nach Anspruch 3 gewonnen werden kann, bei der Herstellung eines Medikaments zur Freisetzung von LH und FSH.

14. Verfahren, das die Herstellung eines Antikörpers, der zur Bindung an ein C-terminales Polypeptid (CTP) fähig ist, so wie es durch das Verfahren nach Anspruch 1 oder 2 gewonnen werden kann, umfaßt.

15. Verfahren nach Anspruch 14, das weiters die Markierung des Antikörpers mit einem nachweisbaren Marker oder seine Insolubilisierung umfaßt.

16. Verfahren, das die Markierung von C-terminalem Polypeptid (CTP), wie es durch das Verfahren nach Anspruch 1 oder Anspruch 2 gewonnen werden kann, oder einer Fusion, wie sie durch das Verfahren nach Anspruch 3 gewonnen werden kann, umfaßt, worin das Polypeptid oder die Fusion mit einem nachweisbaren Marker markiert wird.

17. Diagnostisches Verfahren, das die Entnahme einer Testprobe und die Bestimmung der CTP-Menge in genannter Probe umfaßt, wobei das CTP jene Aminosäuresequenz besitzt, die bei Aminosäuren 14 bis 66 in Fig.1 dargestellt ist, oder eine Mutation mit Ausnahme anderer Fusionen als mit C-terminalem lys-lys-ile, oder ein Fragment genannter dargestellter Sequenz oder eine Mutation eines genannten Fragments ist, wobei das Fragment oder die Mutation mit CTP, so wie es bei Aminosäuren 14 bis 66 in Fig.1 dargestellt ist, immunologisch kreuzreaktiv sind oder im wesentlichen die gleiche biologische Aktivität haben.

18. Verfahren nach einem der Ansprüche 1 bis 3 zur synthetischen Herstellung eines Polypeptids oder einer Fusion, wobei das Verfahren umfaßt:
(a) Transformieren einer Wirtszelle mit einem Vektor, von dem bekannt ist, daß er für das Polypeptid oder die Fusion kodiert; und
(b) Kultivierung der Zelle.

19. Verfahren nach Anspruch 18, worin das Polypeptid CTP, das jene Aminosäuresequenz besitzt, die bei Aminosäuren 14 bis 66 in Fig.1 dargestellt ist, oder eine Fusion genannter dargestellter Sequenz mit einem anderen Polypeptid ist.

20. Verfahren nach Anspruch 19, worin die Fusion ein an den N-Terminus von CTP gebundenes mikrobielles Polypeptid, oder das an den C-Terminus von CTP gebundene Tripeptid lys-lys-ile ist.

21. Verfahren nach Anspruch 20, worin das mikrobielle Polypeptid ein bakterielles Signal ist, das mit CTP zur Sekretion von CTP operabel verbunden ist.

22. Verfahren, das die Herstellung eines DNA-Isolats umfaßt, das für das Polypeptid oder die Fusion, wie sie durch das Verfahren nach einem der Ansprüche 1 bis 3 gewonnen werden können, kodiert, mit der Maßgabe, daß die genannte DNA nicht für eine CTP-Fusion mit LHRH kodiert.

23. Verfahren nach Anspruch 22, worin genannte DNA operabel in einen replizierbaren Vektor ligiert wird.

24. Wirtszelle, die mit dem Vektor nach Anspruch 23 transformiert ist.

25. Verwendung eines Antikörpers, wie er durch das Verfahren nach Anspruch 14 gewonnen werden kann, bei der Herstellung einer Zusammensetzung zur passiven Immunisierung gegen CTP.

## Revendications (Revendications pour l'(les) Etat(s) contractant(s) suivant(s): BE, CH, DE, FR, GB, IT, LI, LU, NL, SE)

1. Polypeptide C-terminal (CTP) ayant la séquence d'amino-acides représentée par les amino-acides 14 à 66 de la figure 1, ou qui est une mutation, à l'exclusion de produits de fusion autres qu'avec un groupe lys-lys-ile C-terminal, ou un fragment de ladite séquence représentée, ou une mutation dudit fragment, le fragment ou la mutation présentant une réactivité croisée immunologique avec le CTP représenté par les amino-acides 14 à 66 de la figure 1 ou présentant une activité biologique pratiquement identique, ledit CTP étant dépourvu de LHRH.

2. Polypeptide suivant la revendication 1, ayant la séquence d'amino-acides représentée par les amino-acides 14 à 66 de la figure 1.

3. Produit de fusion de CTP autre qu'avec la LHRH, le produit de fusion comprenant un CTP ayant la séquence d'amino-acides représentée par les amino-acides 14 à 66 de la figure 1, ou qui est une mutation ou un fragment de ladite séquence représentée, ou une mutation d'un tel fragment, le fragment ou la mutation présentant une réactivité croisée immunologique avec le CTP représenté par les amino-acides 14 à 66 de la figure 1 ou ayant une activité biologique pratiquement identique.

4. Conjugué immunogène d'un CTP suivant la revendication 1 ou la revendication 2 ou d'un produit de fusion de CTP suivant la revendication 3.

5. Conjugué immunogène suivant la revendication 4, dans lequel le CTP ou le produit de fusion de CTP est réticulé par covalence avec une protéine immunogène.

6. Composition comprenant : un polypeptide C-terminal (CTP) suivant la revendication 1 ou la revendication 2, un produit de fusion suivant la revendication 3 ou un conjugué immunogène suivant la revendication 4 ou la revendication 5, pour une utilisation pharmaceutique.

7. Composition suivant la revendication 6, qui comprend une barrière semi-perméable à travers laquelle le polypeptide diffuse à une vitesse prédéterminée.

8. Composition suivant la revendication 6, dans un vaccin.

9. Utilisation d'un conjugué immunogène suivant la revendication 4 pour la production d'anticorps contre le CTP.

10. Utilisation suivant la revendication 9, pour la production d'anticorps contre le CTP chez un animal laitier.

11. Utilisation d'un polypeptide C-terminal (CTP), la séquence d'amino-acides dudit CTP étant représentée par les amino-acides 14 à 66 de la figure 1 ou ledit CTP étant une mutation ou un fragment de ladite séquence représentée ou bien une mutation d'un tel fragment, le fragment ou la mutation présentant une réactivité croisée immunologique avec le CTP représenté par les amino-acides 14 à 66 de la figure 1 ou ayant une activité bioloqique pratiquement identique, ou d'un produit de fusion dudit CTP, dans la préparation d'un médicament destiné à l'inhibition de la libération de prolactine.

12. Utilisation suivant la revendication 11, dans laquelle le médicament est destiné au traitement d'un hypogonadisme dépendant d'une hyperprolactinémie, ou d'une aménorrhée, ou bien de tumeurs dépendant de la prolactine.

13. Utilisation d'un polypeptide C-terminal (CTP) suivant la revendication 1 ou la revendication 2, ou d'un produit de fusion suivant la revendication 3 dans la préparation d'un médicament destiné à la libération de LH et de FSH.

14. Anticorps capable de se lier à un polypeptide C-terminal (CTP) suivant la revendication 1 ou la revendication 2.

15. Anticorps suivant la revendication 14, marqué avec un marqueur détectable ou bien insolubilisé.

16. Polypeptide C-terminal (CTP) suivant la revendication 1 ou la revendication 2, ou produit de fusion de CTP suivant la revendication 3 marqué avec un marqueur détectable.

17. Méthode de diagnostic comprenant l'obtention d'un échantillon d'essai et la détermination de la quantité de CTP dans ledit échantillon, ledit CTP ayant la séquence d'amino-acides représentée par les amino-acides 14 à 66 de la figure 1, ou étant une mutation, à l'exclusion de produits de fusion autres qu'avec un groupe lys-lys-ile C-terminal, ou un fragment de ladite séquence représentée, ou bien une mutation d'un tel fragment, le fragment ou la mutation présentant une réactivité croisée immunologique avec le CTP représenté par les amino-acides 14 à 66 de la figure 1 ou présentant pratiquement la même activité biologique.

18. Procédé de synthèse d'un polypeptide suivant la revendication 1 ou la revendication 2, ou d'un produit de fusion suivant la revendication 3 ; le procédé comprenant :
(a) la transformation d'une cellule hôte avec un vecteur connu pour coder pour le polypeptide ou le produit de fusion ; et
(b) la culture de la cellule.

19. Procédé suivant la revendication 18, dans lequel le polypeptide est un CTP ayant la séquence d'amino-acides représentée par les amino-acides 14 à 66 de la figure 1, ou est un produit de fusion de ladite séquence représentée avec un autre polypeptide.

20. Procédé suivant la revendication 19, dans lequel le produit de fusion est un polypeptide microbien lié à l'extrémité N-terminale du CTP, ou bien le tripeptide lys-lys-ile lié à l'extrémité C-terminale du CTP.

21. Procédé suivant la revendication 20, dans lequel le polypeptide microbien est un signal bactérien lié de manière fonctionnelle au CTP pour la sécrétion du CTP.

22. Isolat d'ADN codant pour le polypeptide suivant l'une quelconque des revendications 1 et 2, le produit de fusion suivant la revendication 3 ou le conjugué suivant la revendication 4, sous réserve que ledit ADN ne code pas pour un produit de fusion du CTP avec la LHRH.

23. ADN suivant la revendication 22, qui est introduit par ligation fonctionnelle dans un vecteur réplicable.

24. Cellule hôte transformée avec le vecteur suivant la revendication 23.

25. Utilisation d'un anticorps suivant la revendication 14, dans la production d'une composition destinée à une immunisation passive contre le CTP.

## Revendications (Revendications pour l'(les) Etat(s) contractant(s) suivant(s): AT)

1. Procédé qui comprend la préparation d'un polypeptide C-terminal (CTP) ayant la séquence d'amino-acides représentée par les amino-acides 14 à 66 de la figure 1, ou qui est une mutation, à l'exclusion de produits de fusion autres qu'avec un groupe lys-lys-ile C-terminal, ou un fragment de ladite séquence représentée, ou bien une mutation d'un tel fragment, le fragment ou la mutation présentant une réactivité croisée immunologique avec le CTP représenté par les amino-acides 14 à 66 de la figure 1 ou bien ayant pratiquement la même activité biologique, ledit CTP étant dépourvu de LHRH.

2. Procédé suivant la revendication 1, dans lequel le polypeptide est le CTP ayant la séquence d'amino-acides représentée par les amino-acides 14 à 66 de la figure 1.

3. Procédé qui comprend la préparation d'un produit de fusion de CTP, autre qu'avec la LHRH, le produit de fusion comprenant un CTP ayant la séquence d'amino-acides représentée par les!amino-acides 14 à 66 de la figure 1, ou qui est une mutation ou un fragment de ladite séquence représentée, ou bien une mutation d'un tel fragment, le fragment ou la mutation présentant une réactivité croisée immunologique avec le CTP représenté par les amino-acides 14 à 66 de la figure 1, ou ayant pratiquement la même activité biologique.

4. Procédé qui comprend la préparation d'un conjugué immunogène d'un CTP, pouvant être obtenu par le procédé suivant la revendication 1 ou la revendication 2, ou bien d'un produit de fusion de CTP pouvant être obtenu par le procédé suivant la revendication 3.

5. Procédé suivant la revendication 4, dans lequel le CTP ou le produit de fusion de CTP est réticulé par covalence à une protéine immunogène.

6. Utilisation d'un CTP pouvant être obtenu par le procédé suivant la revendication 1 ou la revendication 2, ou d'un produit de fusion pouvant être obtenu par le procédé suivant la revendication 3, ou bien d'un conjugué immunogène pouvant être obtenu par le procédé suivant la revendication 4 ou la revendication 5 dans la préparation d'un produit pharmaceutique.

7. Utilisation suivant la revendication 6, dans laquelle le produit pharmaceutique comprend une barrière semi-perméable à travers laquelle le polypeptide diffuse à une vitesse prédéterminée.

8. Utilisation suivant la revendication 6, dans laquelle le produit pharmaceutique est un vaccin.

9. Utilisation d'un conjugué immunogène pouvant être obtenu par le procédé suivant la revendication 4 pour la production d'anticorps contre le CTP.

10. Utilisation suivant la revendication 9 pour la production d'anticorps contre le CTP chez un animal laitier.

11. Utilisation d'un polypeptide C-terminal (CTP), la séquence d'amino-acides dudit CTP étant telle que représentée par les amino-acides 14 a 66 de la figure 1 ou bien ledit CTP étant une mutation ou un fragment de ladite séquence représentée, ou une mutation d'un tel fragment, le fragment ou la mutation présentant une réactivité croisée immunologique avec le CTP représenté par les amino-acides 14 à 66 de la figure 1 ou ayant pratiquement la même activité biologique ; ou bien d'un produit de fusion dudit CTP ; dans la préparation d'un médicament destiné à l'inhibition de la libération de prolactine.

12. Utilisation suivant la revendication 11, dans laquelle le médicament est destiné au traitement de l'hypogonadisme dépendant d'une hyperprolactinémie, ou de l'aménorrhée, ou bien de tumeurs dépendant de la prolactine.

13. Utilisation d'un polypeptide C-terminal (CTP) pouvant être obtenu par le procédé suivant la revendication 1 ou la revendication 2, ou bien d'un produit de fusion pouvant être obtenu par le procédé suivant la revendication 3 dans la préparation d'un médicament destiné à la libération de LH et de FSH.

14. Procédé qui comprend la préparation d'un anticorps capable de se lier à un polypeptide C-terminal (CTP) pouvant être obtenu par le procédé suivant la revendication 1 ou 2.

15. Procédé suivant la revendication 14, comprenant en outre le marquage de l'anticorps avec un marqueur détectable ou son insolubilisation.

16. Procédé qui comprend le marquage d'un polypeptide C-terminal (CTP) pouvant être obtenu par le procédé suivant la revendication 1 ou la revendication 2, ou bien d'un produit de fusion pouvant être obtenu par le procédé suivant la revendication 3, dans lequel le polypeptide ou le produit de fusion est marqué avec un marqueur détectable.

17. Méthode de diagnostic comprenant l'obtention d'un échantillon d'essai et la détermination de la quantité de CTP dans ledit échantillon, le CTP ayant la séquence d'amino-acides représentée par les amino-acides 14 à 66 de la figure 1, ou étant une mutation, à l'exclusion de produits de fusion autres qu'avec un groupe lys-lys-ile C-terminal, ou un fragment de ladite séquence représentée, ou bien une mutation d'un tel fragment, le fragment ou la mutation présentant une réactivité croisée immunologique avec le CTP représenté par les amino-acides 14 à 66 de la figure 1 ou ayant pratiquement la même activité biologique.

18. Procédé suivant l'une quelconque des revendications 1 à 3 pour la synthèse d'un polypeptide ou d'un produit de fusion, le procédé comprenant :
(a) la transformation d'une cellule hôte avec un vecteur connu pour coder pour le polypeptide ou le produit de fusion ; et
(b) la culture de la cellule.

19. Procédé suivant la revendication 18, dans lequel le polypeptide est un CTP ayant la séquence d'amino-acides représentée par les amino-acides 14 à 66 de la figure 1, ou bien est un produit de fusion de ladite séquence représentée avec un autre polypeptide.

20. Procédé suivant la revendication 19, dans lequel le produit de fusion est un polypeptide microbien lié à l'extrémité N-terminale du CTP, ou bien le tripeptide lys-lys-ile lié à l'extrémité C-terminale du CTP.

21. Procédé suivant la revendication 20, dans lequel le polypeptide microbien est un signal bactérien lié de manière fonctionnelle au CTP pour la sécrétion du CTP.

22. Procédé qui comprend la préparation d'un isolat d'ADN codant pour le polypeptide ou le produit de fusion pouvant être obtenu par le procédé suivant l'une quelconque des revendications 1 à 3, sous réserve que ledit ADN ne code pas pour un produit de fusion du CTP avec la LHRH.

23. Procédé suivant la revendication 22, dans lequel l'ADN est introduit par ligation fonctionnelle dans un vecteur réplicable.

24. Cellule hôte transformée avec le vecteur suivant la revendication 23.

25. Utilisation d'un anticorps pouvant être obtenu par le procédé suivant la revendication 14 dans la production d'une composition destinée à une immunisation passive contre le CTP.
